# EUROPEAN PATENT SPECIFICATION

(11) **EP 4 256 570 B1**
(45) Date of publication and mention of the grant of the patent: **08.10.2025**
(21) Application number: 21824359.0
(22) Date of filing: 02.12.2021
(51) Int. Cl.: G16H 20/10, G16H 20/30, G16H 20/60, G16H 50/20, G16H 50/50

(54) **IMPUTATION OF DATA USING CONTEXTUAL INFORMATION**
ZUSCHREIBUNG VON DATEN UNTER VERWENDUNG VON KONTEXTUELLEN INFORMATIONEN
IMPUTATION DE DONNÉES À L'AIDE D'INFORMATIONS CONTEXTUELLES

(30) Priority: 03.12.2020 EP 20383057
(43) Date of publication of application: 11.10.2023
(73) Proprietor: F. Hoffmann-La Roche AG, 4070 Basel (CH); Roche Diabetes Care GmbH, 68305 Mannheim (DE)
(72) Inventor: DAJANI, Samer M., Indianapolis, Indiana 46250 (US); GEJDOS, Igor, Indianapolis, Indiana 46250 (US); MANOHAR, Chinmay, Indianapolis, Indiana 46250 (US); RODRIGUES, Joao, 08174 Sant Cugat del Vallès, Barcelona (ES)
(74) Representative: J A Kemp LLP
(86) International application number: PCT/EP2021/083933
(87) International publication number: WO 2022/117713

(56) References cited:
- US-A1- 2019 252 079
- US-A1- 2020 176 121

## Description

### TECHNICAL FIELD

The present invention relates generally to methods and systems for using predictive learning in diabetes disease progression analysis and treatment.

### BACKGROUND

Devices are used to monitor one or more parameters associated with a user during a testing period. The user is a person with diabetes (user), and the testing period includes monitoring blood glucose levels and other data. The monitored blood glucose levels and other data is used to monitor progression of the diabetes disease and therapy optimization. Strict compliance and diligent capture of the blood glucose levels and other data during the testing period is critical for accurately determine the state of their diabetes disease and any therapy adjustments needed. Unfortunately, some users (e.g., pre-diabetics and non-insulin dependent Type 2 diabetics) are not accustomed to habitual testing and recording of data and are thus challenged when tasked with structured testing periods. And, one or more instances of data may be missed during the testing period. When one or more instances of data is missed during the testing period, the testing period needs to be extended such that a complete and reliable set of data is available for disease progression and therapy optimization analysis.

US 2020/0176121 A1 describes various systems and methods. One or more of the methods disclosed uses machine learning algorithms to predict biophysical responses from biophysical data, such as heart rate monitor data, food logs, or glucose measurements. Biophysical responses may include behavioral responses. Additional systems and methods extract nutritional information from food items by parsing strings containing names of food items.

In US 2019/0252079 A1, systems and methods are described to provide guidance to a user regarding management of a physiological condition such as diabetes. The determination may be based upon a patient glucose concentration data sensed by a glucose concentration sensor. A host state change associated with the host glucose concentration data may be determined. A guidance message based at least in part on the host state change may also be determined. The guidance message may be delivered through a user interface.

### SUMMARY

A system and a method are disclosed for reliably capturing structured testing data to ensure accurate and compliant data collection supplemented with intelligent imputation of missed values based on contextual information and predictive learning. A user device *(e.g.,* a mobile device) is implemented to provide reliable capture of blood glucose data, dietary intake data, medication data, and activity data from a user *(e.g.,* a patient with diabetes (PwD)). The user device receives blood glucose data from a blood glucose monitor. The blood glucose monitor is a continuous glucose monitor (CGM) or a spot-monitoring blood glucose (SMBG) meter. The blood glucose data includes a plurality of blood glucose levels of the user that are measured during a testing period. The testing period includes one or more days (*e.g*., three days). The user device receives a testing period data associated with the user during testing period. The testing period data includes dietary intake data associated with a plurality of meals consumed by the user during the testing period, medication data associated with a plurality of medication doses taken by the user during the testing period, and activity data associated with a plurality of pre-scheduled activities for the user during the testing period.

The user device determines that a portion of the data is missing based on an analysis of the received testing period data. The analysis of the received data includes an analysis of one or more blood glucose levels of the plurality of glucose levels and an analysis of the dietary intake data, the medication data, and the activity data. The analysis of the one or more blood glucose levels includes determining that a blood glucose level is greater than a pre-defined blood glucose threshold. The pre-defined blood glucose threshold is determined based on one or more of an expected blood glucose level associated with one or more of a carbohydrate content of the meal, a glycemic profile of the meal, a dynamic fingerprint associated with a medicine of the medication dose, a type of the pre-scheduled activity, a length of the pre-scheduled activity, or a pre-determined blood glucose level increase associated with the user. The pre-defined blood glucose threshold is determined based on a generic population-based glucose model in response to the meal, pre-scheduled activity, or medication dose being on a first day of the testing period. The pre-defined blood glucose threshold is determined based on a time-weighted blood glucose model in response to the meal, pre-scheduled activity, or medication dose being on a second or third day of the testing period. The time-weighted blood glucose model is developed using blood glucose data of the user received on the first day of the testing period.

The user device imputes the missing portion of the data with substitute dietary intake data in response to determining that the missing portion of the data comprises dietary intake data associated with a meal of the plurality of meals and determining that the user consumed the meal. The user device uses contextual information to determine the substitute dietary intakee data. The user device determines that the missing portion of the data includes dietary intake data includes analysis of one or more scheduled meals. The user device imputes the missing portion of the data with substitute medication data in response to determining that the missing portion of data comprises medication data associated with a medication dose of the plurality of medication doses and determining that the user took the medication dose. The user device determines that the missing portion of data comprises medication data comprises analysis of one or more scheduled medication doses. The user device imputes the missing portion of the data with substitute activity data in response to determining that the missing portion of data comprises activity data associated with a pre-scheduled activity of the plurality of pre-scheduled activities and determining that the user participated in the pre-scheduled activity. The user device determines that the missing portion of the data includes activity data includes analysis of one or more pre-scheduled activities.

The substitute data, such as the substitute dietary intake data, the substitute activity data, and the substitute medication data, may be determined by using contextual information, such as at least one blood glucose model, comprising pre-determined data on at least one reference blood glucose level associated with one or more of a dietary intake, an activity, and a medication intake. The substitute data, such as the substitute dietary intake data, the substitute activity data, and the substitute medication data, may specifically be based on one or more of a generic population-based blood glucose model and a time-weighted blood glucose model, wherein the population-based blood glucose model may specifically comprise pre-determined information on the blood glucose level of at least one exemplary individual in response to one or more of a dietary intake, an activity, and a medication intake, and wherein the time-weighted blood glucose model may specifically comprise pre-determined information on the blood glucose level of the user to one or more of a dietary intake, an activity, and a medication intake.

The substitute dietary intake data, the substitute activity data, and the substitute medication data are based on a generic population-based blood glucose model in response to the missing portion of the data being associated with a first time period on a first day of the plurality of days.

The generic population-based blood glucose model may be or may comprise at least one predictive blood glucose level response. In other words, the generic population-based blood glucose model may be or may comprise information on how one or more of the dietary intake, such as a meal, i.e. a pre-packaged meal, the pre-scheduled activity, and the medication dose, i.e. a pre-packaged medication dose, influences a blood glucose level of a reference individual, such as of an average and/or representative patient. Thus, as an example, the generic population-based blood glucose model may comprise pre-determined data, such as one or more standard values and/or a calculation procedure, i.e. at least one algorithm, regarding an average blood glucose development according to one or more of the dietary intake, the pre-scheduled activity and the medication dose. In particular, as a simple example, the generic population-based blood glucose model may be or may comprise one or more sets of data, i.e. a data table or matrix, assigning a specific blood glucose level with one or more of a dietary intake, an activity, i.e. a pre-scheduled activity, and a medication intake, such as a medication dose intake, or vice versa. Specifically, the pre-determined data, i.e. dietary intake data, activity data or medication data, associated with the blood glucose level, may then be used as substitute data, i.e. as substitute dietary intake data, substitute activity data and/or substitute medication data.

The generic population-based blood glucose model may be or may comprise a trained model, such as a mathematical model, which was trained on at least one training data set, e.g. a set of predetermined training data, by using one or more of machine learning, deep learning, neural networks, or other form of artificial intelligence. As an example, the generic population-based blood glucose model may be a trained model comprising at least one model selected from the group consisting of: a linear regression model, e.g. comprising transformed features, such as log-transformed or polynomial; at least one non-linear Artificial Neural Network (ANN), in particular at least one deep learning architecture such as Convolutional NN, Recurrent NN, Long Short Term Memory NN, and the like; at least one Support Vector Machine (SVM); at least one kernel based method; Tree regression; Random Forest.

The generic population-based blood glucose model may specifically be or may comprise a trained model, trained with a training data set comprising historical experimental data such as previously determined data, e.g. previously determined blood glucose levels. Specifically, the generic population-based blood glucose model may be or may comprise predictive blood glucose level responses using data from persons other than the user. As an example, the training data for the generic population-based blood glucose model may be obtained by measuring and linking the blood glucose levels of a plurality of diabetes patients with regard to internal and external factors and/or influences. In particular, the blood glucose levels may be measured and/or monitored after the patients are being subjected to external influences, such as physical exercise and nutrition, i.e. food, beverages, medications, or other orally supplied intake. Further, internal factors may be determined, such as the diabetes patients' personal data, e.g. age, gender, weight, duration of disease, disease progression, type of medication, general state of health, or other personal factors and/or parameters. Other data may additionally be obtained, such as environmental information, i.e. weather data or the like. The respective measured blood glucose levels may be used as training data, wherein the training data may be labeled with the respective potential influences, such as the external influences and/or the internal factors and/or additional information on the circumstances under which the training data were obtained. All the information may be labeled and linked, i.e. in a subsequent step to measuring and determining the data, with the blood glucose levels, thereby, as an example, obtaining the set of training data for training the generic population-based blood glucose model. Other ways of obtaining the training data for the generic population-based blood glucose model may be feasible.

The substitute dietary intake data, the substitute activity data, and the substitute medication data are based on a time-weighted blood glucose model developed using blood glucose data of the user received on the first day of the plurality of days in response to the missing portion of the data being associated with a second time period on a second or third day of the plurality of days.

The time-weighted blood glucose model may be or may comprise at least one predictive blood glucose level response further based on testing period data of the user. In other words, the time-weighted blood glucose model may be based on the generic population-based blood glucose model updated by information on how one or more of the dietary intake, such as a meal, i.e. a pre-packaged meal, the pre-scheduled activity and the medication dose, i.e. a pre-packaged medication dose, influenced a blood glucose level of the user on the first day, and possibly further days after the first day, of the plurality of days. Thus, as an example, the time-weighted blood glucose model may comprise pre-determined data, such as one or more values, regarding the blood glucose development of the user according to one or more of the dietary intake, the pre-scheduled activity and the medication dose, as measured and/or received on the first day. In particular, as a simple example, the time-weighted blood glucose model may be or may comprise one or more sets of data, i.e. a data table or matrix, assigning a specific blood glucose level of the user with one or more of the user's dietary intake, the user's activity, i.e. a pre-scheduled activity, and the user's medication intake, such as a medication dose, taken and/or performed by the user on the first day, or vice versa. As an example, the time-weighted blood glucose model may be time-weighted such that blood glucose level responses of the user closest a current day of the testing period are assigned a higher relative weight. Specifically, the pre-determined data, i.e. the user's dietary intake data, activity data or medication data, associated with the user's blood glucose level on the first day, may then be used as substitute data for the subsequent days, i.e. as substitute dietary intake data, substitute activity data and/or substitute medication data. In particular, an accuracy of the time-weighted blood glucose model may evolve and improve with each passing day, i.e. by collecting and adding user-specific blood glucose and intake or performance data each day. Thus, on the third day, the time-weighted blood glucose model may be or may comprise data assigning the blood glucose level of the user with one or more of the user's dietary intake, the user's activity and the user's medication intake of both the first day and the second day, i.e. with an average value between the two days.

The time-weighted blood glucose model may be or may comprise a trained model, such as a mathematical model, which was trained on at least one training data set comprising information on the user, e.g. a set of predetermined user specific training data, by using one or more of machine learning, deep learning, neural networks, or other form of artificial intelligence. As an example, the time-weighted blood glucose model may be a trained model comprising at least one model selected from the group consisting of: a linear regression model, e.g. comprising transformed features, such as log-transformed or polynomial; at least one non-linear Artificial Neural Network (ANN), in particular at least one deep learning architecture such as Convolutional NN, Recurrent NN, Long Short Term Memory NN, and the like; at least one Support Vector Machine (SVM); at least one kernel based method; Tree regression; Random Forest.

In particular, the time-weighted blood glucose model may be or may comprise a trainable model, such as a trained model that can be further trained and/or updated based on additional training data generated from the user, e.g. continuously being generated by the user. Thus, the time-weighted blood glucose model may be or may comprise a trained model, i.e. similar to the generic population-based blood glucose model, but trained with information on the blood glucose of the user, i.e. with a training data set generated from the user, and further being trained and/or updated based on additional data from the user. Specifically, the time-weighted blood glucose model may be or may comprise predictive blood glucose level responses using the user's testing period data from previous days of the testing period. As an example, the time-weighted blood glucose model may be or may comprise the generic population-based blood glucose model updated by using the testing period data from previous days of the testing period. The time-weighted blood glucose model may specifically be or may comprise a trainable model trained and updated with training data generated from the user, such as blood glucose levels of the user as previously determined and as continuing to be determined, specifically the received blood glucose data from the blood glucose monitor and the testing period data, i.e. the dietary intake data, the medication data and the activity data. In particular, the respective measured blood glucose levels of the user may be used as training data, wherein the training data may be labeled with the respective potential influences, such as the respective testing period data, i.e. the dietary intake data, the medication data and the activity data, under which the training data were obtained. All the information may be labeled and linked, i.e. in a subsequent step to measuring and determining the training period data, with the blood glucose levels of the user, thereby, as an example, obtaining the set of training data from the user for training the time-weighted blood glucose model. Other ways of obtaining the training data for the time-weighted blood glucose model may be feasible.

The user device identifies, using the substitute dietary intake data, the substitute medication data, or the substitute activity data, a progression or a regression in a diabetic condition associated with the user. The user device adjusts a configuration of an insulin pump to increase or reduce the supply of insulin to the user in response to the progression or the regression in the diabetic condition.

The user device calculates a confidence level associated with the substitute dietary intake data, the substitute medication data, or the substitute activity data. The confidence levels for each instance of substitute data imputation on a day of the testing period are aggregated to determine a daily aggregate confidence level. The user device extends the testing period in response to the daily aggregate confidence level being below a pre-defined confidence threshold. The user device adds the substitute dietary intake data, the substitute medication data, or the substitute activity data to the testing period data when the confidence level is greater than the pre-defined confidence threshold. The user device updates a learning model associated with the user using the testing period data.

The user device extends the testing period in response to determining that the user did not consume the meal based on an analysis of one or more of the plurality of glucose levels proximate to a first time period associated with the meal. The user device extends the testing period in response to determining that the user did not take the medication dose based on an analysis of one or more of the plurality of glucose levels proximate to a second time period associated with the medication dose. The user device extends the testing period in response to determining that the user did not participate in the pre-scheduled activity based on an analysis of one or more of the plurality of glucose levels proximate to a third time period associated with the pre-scheduled activity. The user device extends the testing period in response to determining that the confidence level is below a pre-determined confidence threshold.

In embodiments, a user device is configured to impute missing dietary intake data with substitute data. The user devices uses contextual information to determine the substitute data. The user device receives blood glucose data from a blood glucose monitor, the blood glucose data comprising a plurality of blood glucose levels of a user that are measured during a testing period. The blood glucose monitor is a CGM or SMBG meter. The user device receives receiving dietary intake data associated with a plurality of meals consumed by the user during the testing period. The dietary intake data comprises macronutrient information for the plurality of meals consumed by the user during the testing period, and wherein the plurality of meals are pre-packaged meals.

The user device determines that a portion of the dietary intake data is missing based on analysis of the received dietary intake data. The missing portion of the dietary intake data is associated with a meal of the plurality of meals. The user device determines that the user consumed the meal based on a blood glucose level of the plurality of blood glucose levels being greater than a pre-defined blood glucose threshold. The blood glucose level is proximate to a time period associated with the meal. The user device imputes the missing portion of the dietary intake data with substitute data in response to determining that the user consumed the meal. The testing period comprises a plurality of days. The substitute data is based on a generic population-based blood glucose model in response to the meal being on a first day of the plurality of days. The substitute data is based on a time-weighted blood glucose model developed using blood glucose data of the user received on the first day of the plurality of days in response to the meal being on a second or third day of the plurality of days. The substitute data enables one or more of disease progression analysis or therapy analysis at an end of the testing period.

The substitute dietary intake data may specifically be determined by using contextual information, such as at least one blood glucose model, i.e. one or more of a generic population-based blood glucose model and a time-weighted blood glucose model, comprising pre-determined data on at least one blood glucose level associated with the dietary intake or at least one similar dietary intake, such as with a previous dietary intake. For example, the dietary intake or the similar dietary intake may be or may comprise an intake of a pre-packaged meal, such as of a meal of known nutrition information. As a simple example, the substitute dietary intake data may be a pre-determined value, such as an average value or a standard value, of dietary intake data. Specifically, as an example, in case the missing portion of the testing period data is being associated with a first time period on a first day of the plurality of days, the substitute dietary intake data may be a value of dietary intake data as suggested and/or predicted by the generic blood glucose model. Additionally or alternatively, as an example, in case the missing portion of the testing period data is being associated with a second time period on a second or third day of the plurality of days, the substitute dietary intake data may be a time-weighted value of dietary intake data as suggested and/or predicted by the time-weighted blood glucose model.

The user device calculates a confidence level of the substitute data imputation. The confidence levels for each instance of substitute data imputation on a day of the testing period are aggregated to determine a daily aggregate confidence level.

The user device extends the testing period in response to determining that the user did not consume the meal based on an analysis of one or more of the plurality of glucose levels proximate to the time period. The analysis of the one or more blood glucose levels proximate to the time period comprises determining that the one or more blood glucose levels proximate to the time period are less than a pre-defined blood glucose threshold. The pre-defined blood glucose threshold is determined based on an expected blood glucose level associated with one or more of a carbohydrate content of the meal, a glycemic profile of the meal, or a pre-determined meal blood glucose level increase associated with the user. The pre-defined blood glucose threshold is determined based on a generic population-based blood glucose model in response to the meal being on a first day of the plurality of days. The pre-defined blood glucose threshold is determined based on a time-weighted blood glucose model in response to the meal being on a second or third day of the plurality of days. The time-weighted blood glucose model is developed using blood glucose data of the user received on the first day of the plurality of days.

The user device extends the testing period in response to determining that the confidence level of the substitute data is below a pre-determined confidence threshold. The testing period is extended in response to the daily aggregate confidence level being below the pre-defined confidence threshold. The user device identifies, using the substitute data, a progression or a regression in a diabetic condition associated with the user. The user device adjusts a configuration of an insulin pump to increase or reduce the supply of insulin to the user in response to the progression or the regression in the diabetic condition.

In embodiments, a user device is configured to impute missing medication data with substitute data. The user device receives blood glucose data from a blood glucose monitor, the blood glucose data comprising a plurality of blood glucose levels of a user that are measured during a testing period. The blood glucose monitor is a CGM or SMBG meter. The user device receives medication data associated with a plurality of medication doses taken by the user during the testing period. The user device determines that a portion of the medication data is missing based on analysis of the received medication data. The missing portion of the medication data is associated with a medication dose of the plurality of medication doses. The user device determines that the user took the medication dose based on a blood glucose level of the plurality of blood glucose levels being greater than a pre-defined blood glucose threshold and an analysis of activity data proximate to the time period associated with the medication dose. For example, the user device determines that the blood glucose level is associated with the medication dose and not associated with a missing portion of activity data. The blood glucose level is proximate to a time period associated with the medication dose.

The user device imputes the missing portion of the medication data with substitute data in response to determining that the user took the medication. The user device uses contextual information to determine the substitute data. The substitute data enables one or more of disease progression analysis or therapy analysis at an end of the testing period. The testing period comprises a plurality of days. The substitute data is based on a generic population-based blood glucose model in response to the medication dose being on a first day of the plurality of days. The substitute data is based on a time-weighted blood glucose model developed using blood glucose data of the user received on the first day of the plurality of days in response to the medication dose being on a second or third day of the plurality of days. The user device calculates a confidence level of the substitute data imputation.

The substitute data, i.e. the substitute medication data, may specifically be determined by using contextual information, such as at least one blood glucose model, i.e. one or more of a generic population-based blood glucose model and a time-weighted blood glucose model, comprising pre-determined data on at least one blood glucose level associated with the medication dose intake or at least one similar medication intake, such as with a previous medication dose intake. For example, the medication dose intake or the similar medication intake may be or may comprise an intake of a pre-packaged medication dose, such as of a medication dose of known dynamic fingerprint, i.e. of known influences on the blood glucose level. As a simple example, the substitute medication data may be a pre-determined value, such as an average value or a standard value, of medication data. Specifically, as an example, in case the missing portion of the testing period data is being associated with a first time period on a first day of the plurality of days, the substitute medication data may be a value of medication data as suggested and/or predicted by the generic blood glucose model. Additionally or alternatively, as an example, in case the missing portion of the testing period data is being associated with a second time period on a second or third day of the plurality of days, the substitute medication data may be a time-weighted value of medication data as suggested and/or predicted by the time-weighted blood glucose model.

The user device extends the testing period in response to determining that the user did not take the medication dose based on an analysis of one or more of the plurality of glucose levels proximate to the time period. The analysis of the one or more blood glucose levels proximate to the time period comprises determining that the one or more blood glucose levels proximate to the time period are less than the pre-defined blood glucose threshold. The pre-defined blood glucose threshold is determined based on a dynamic fingerprint associated with a medicine of the medication dose or a pre-determined medication blood glucose level increase associated with the user. The dynamic fingerprint comprises one or more of an onset time, an acting time, or a drop time. The pre-defined blood glucose threshold is determined based on a generic population-based blood glucose model in response to the medication dose being on a first day of the plurality of days. The pre-defined blood glucose threshold is determined based on a time-weighted blood glucose model in response to the medication dose being on a second or third day of the plurality of days, the time-weighted blood glucose model developed using blood glucose data of the user received on the first day of the plurality of days.

The user device extends the testing period in response to determining that the confidence level of the substitute data is below a pre-determined confidence threshold. The confidence levels for each instance of substitute data imputation on a day of the testing period are aggregated to determine a daily aggregate confidence level, and wherein the testing period is extended in response to the daily aggregate confidence level being below the pre-defined confidence threshold. The user device identifies, using the substitute data, a progression or a regression in a diabetic condition associated with the user. The user device adjusts a configuration of an insulin pump to increase or reduce the supply of insulin to the user in response to the progression or the regression in the diabetic condition.

In embodiments, a user device is configured to impute missing activity data with substitute data. The user device receives blood glucose data from a blood glucose monitor, the blood glucose data comprising a plurality of blood glucose levels of a user that are measured during a testing period. The blood glucose monitor is a CGM or SMBG meter. The user device receives activity data associated with a plurality of pre-scheduled activities for the user during the testing period. The user device determines that a portion of the activity data is missing based on analysis of the received activity data. The missing portion of the activity data is associated with a pre-scheduled activity of the plurality of pre-scheduled activities. The user device determines that the user participated in the pre-scheduled activity based on a blood glucose level of the plurality of blood glucose levels being greater than a pre-defined blood glucose threshold. The blood glucose level is proximate to a time period associated with the pre-scheduled activity. The user device determines that the user participated in the pre-scheduled activity based on analysis of medication data proximate to the time period associated with the pre-scheduled activity. The user device determines that the blood glucose level is associated with the pre-scheduled activity and not associated with a missing portion of medication data.

The user device imputes the missing portion of the activity data with substitute data in response to determining that the user participated in the pre-scheduled activity. The substitute data enables one or more of disease progression analysis or therapy analysis at an end of the testing period. The testing period comprises a plurality of days. The substitute data is based on a generic population-based blood glucose model in response to the pre-scheduled activity being on a first day of the plurality of days. The substitute data is based on a time-weighted blood glucose model developed using blood glucose data of the user received on the first day of the plurality of days in response to the pre-scheduled activity being on a second or third day of the plurality of days.

The substitute data, i.e. the substitute activity data, may specifically be determined by using contextual information, such as at least one blood glucose model, i.e. one or more of a generic population-based blood glucose model and a time-weighted blood glucose model, comprising pre-determined data on at least one blood glucose level associated with the pre-scheduled activity or at least one similar activity, such as with a previously performed activity. For example, the pre-scheduled activity or the similar activity may have a known influence on the blood glucose level. As a simple example, the substitute activity data may be a pre-determined value, such as an average value or a standard value, of pre-scheduled activity data. Specifically, as an example, in case the missing portion of the testing period data is being associated with a first time period on a first day of the plurality of days, the substitute activity data may be a value of activity data as suggested and/or predicted by the generic blood glucose model. Additionally or alternatively, as an example, in case the missing portion of the testing period data is being associated with a second time period on a second or third day of the plurality of days, the substitute activity data may be a time-weighted value of activity data as suggested and/or predicted by the time-weighted blood glucose model.

The user device calculates a confidence level of the substitute data imputation. The user device extends the testing period in response to determining that the user did not participate in the pre-scheduled activity based on an analysis of one or more of the plurality of glucose levels proximate to the time period. The analysis of the one or more blood glucose levels proximate to the time period comprises determining that the one or more blood glucose levels proximate to the time period are less than the pre-defined blood glucose threshold. The pre-defined blood glucose threshold is determined based on one or more of a type of the pre-scheduled activity, a length of the pre-scheduled activity, or a pre-determined activity blood glucose level increase associated with the user. The pre-defined blood glucose threshold is determined based on a generic population-based blood glucose model in response to the activity being on a first day of the plurality of days. The pre-defined blood glucose threshold is determined based on a time-weighted blood glucose model in response to the activity being on a second or third day of the plurality of days, the time-weighted blood glucose model developed using blood glucose data of the user received on the first day of the plurality of days.

The user device extends the testing period in response to determining that the confidence level of the substitute data is below a pre-determined confidence threshold. The confidence levels for each instance of substitute data imputation on a day of the testing period are aggregated to determine a daily aggregate confidence level, and wherein the testing period is extended in response to the daily aggregate confidence level being below the pre-defined confidence threshold. The user device identifies, using the substitute data, a progression or a regression in a diabetic condition associated with the user. The user device adjusts a configuration of an insulin pump to increase or reduce the supply of insulin to the user in response to the progression or the regression in the diabetic condition.

### BRIEF DESCRIPTION OF THE DRAWINGS

FIG. 1 is a perspective view of a representative environment for monitoring or treating a diabetic condition.
FIG. 2 shows a flowchart of an example process for using a structured testing period to analyze disease progression and optimize therapy.
FIG. 3 is a flowchart of an example process for reliable capture of data during a structured testing period.
FIG. 4 shows a flowchart of an example process for imputation of dietary data during a structured testing period.
FIG. 5 shows a flowchart of an example process for imputation of activity data during a structured testing period.
FIG. 6 shows a flowchart of an example process for imputation of medication data during a structured testing period.
FIG. 7 shows a flowchart of an example process for imputation of blood glucose data.
FIG. 8 shows a flowchart of an example process for imputation of a fasting blood glucose level.
FIG. 9 is a block diagram of an example blood glucose monitoring device.
FIG. 10 is a block diagram of an example blood glucose measuring (BGM) device.
FIG. 11 is a block diagram illustrating an example of an insulin pump.
FIG. 12 is a block diagram of an example computing device.
FIG. 13 is a block diagram of an example smart plate.

### DETAILED DESCRIPTION

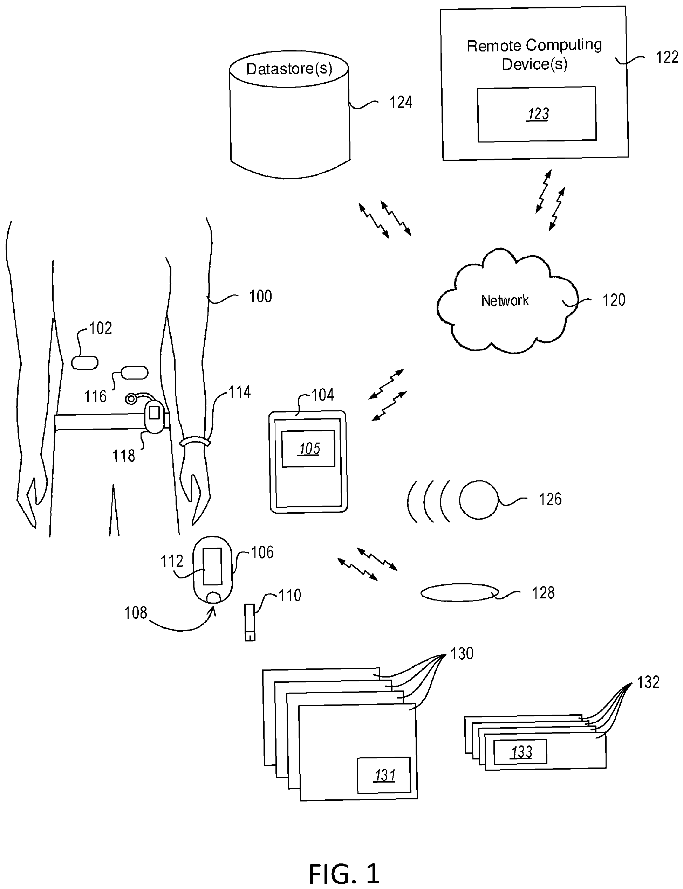

FIG. 1 is a perspective view of a representative environment for monitoring or treating a diabetic condition. As shown in FIG. 1, a user 100 with diabetes uses one or more blood glucose monitoring devices to help monitor or treat the diabetic condition. The diabetic condition includes a metabolic syndrome, pre-diabetes, type 1 diabetes, type 2 diabetes, or gestational diabetes. The user 100 may be in an extreme diabetic state, such as hypoglycemia or hyperglycemia, when the blood glucose level of the user 100 is above or below a threshold blood glucose level. In the embodiment of FIG. 1, the user 100 uses a blood glucose monitoring device to monitor blood glucose levels.

As used herein, the term "blood glucose monitoring device" refers to any device that detects and reports a level of glucose in the blood of the user, either through direct measurement of the blood or through an indirect detection process. A blood glucose level is also referred to as a blood sugar level. Examples of blood glucose monitoring devices include, but are not strictly limited to, continuous glucose monitoring devices, flash glucose monitoring devices, and blood glucose meters that provide a single measurement of blood glucose levels from a blood sample in a "spot" monitoring process. FIG. 1 depicts examples of blood glucose monitoring devices that are described in more detail below.

In some embodiments, the blood glucose monitoring device is a continuous glucose monitor (CGM) 102. The CGM 102 includes a subcutaneous sensor that is used to sense and monitor the amount of glucose in interstitial fluid of the user 100. The CGM 102 includes a transmitting device that is located directly over the sensor that wirelessly powers the data transfer from the sensor. The CGM 102 periodically communicates data indicating the blood glucose levels of the user 100 to an external device, such as a mobile device 104, for computing or storing the blood glucose levels of the user 100.

As used herein, the term "mobile device" refers to any mobile electronic device that is capable of moving with a user as the user changes locations. Example mobile devices include mobile phones, smartphones, wearable devices, tablets, laptops, notebook computers, personal digital assistants (PDAs), and any other mobile electronic device that is capable of moving with a user. Some embodiments of the mobile device incorporate the blood glucose monitor into an integrated device.

Some embodiments of the mobile device 104 operate as a CGM controller device. Though the mobile device 104 is provided as an example of a device with which the CGM 102 communicates, the CGM 102 may communicate with other dedicated CGM controller devices for providing similar functionality that is described herein for the mobile device 104. The CGM 102 processes the blood glucose data to enable disease progression analysis and therapy analysis. The blood glucose data is processed at the mobile device 104, or other CMG controller device, an alert indicator is communicated to the CGM 102.

In some embodiments, the blood glucose monitoring is performed by flash glucose monitoring (FGM). The FGM includes a subcutaneous sensor 103 that is used to sense and monitor the amount of glucose in interstitial fluid of the user 100. A separate reader device, such as the mobile device 104 or another reader device, receives the blood glucose information from the sensor when the device is within the RF range of the sensor 103. The sensor 103 transmits an instantaneous blood glucose level or a graphical trend of the blood glucose level to the reader device for display.

In some embodiments, the CGM 102 is a short-term disposable CGM device. The short-term disposable CGM device is activated at the beginning of the testing period and measures blood glucose levels of the user during the testing period. In examples, the short-term disposable CGM device sends the blood glucose level measurements to the mobile device 104. In examples, the short-term disposable CGM device sends the blood glucose level measurements to a remote computing device 122. The short-term disposable CGM device is disposed of following completion of the testing period.

In some embodiments, the CGM 102 is blinded such that the blood glucose level measurements are not presented to the user during the testing period. Users tend to alter their behavior when they can see their blood glucose level measurements.

In some embodiments, the CGM 102 does not communicate with the mobile device 104. Instead, the CGM 102 stores the blood glucose level measurements until the end of the testing period. In examples, the CGM is configured to transfer the stored blood glucose level measurements to the remote computing device 122.

In some embodiments, the user uses a blood glucose monitor (BGM) 106 *(e.g.,* a spot-monitoring blood glucose (SMBG)) as a blood glucose monitoring device to monitor blood glucose levels. The BGM 106 includes port 108 that receives a blood glucose measurement strip 110. The user 100 deposits a sample of blood on the blood glucose measurement strip 110. The BGM 106 analyzes the sample and measure the blood glucose level in the sample. The blood glucose level measured from the sample is displayed on a display 112 of the BGM 106 or communicated to an external device, such as the mobile device 104.

The blood glucose level measured by the BGM 106 or computed using data received from the CGM 102 is used to treat the diabetic condition of the user 100. The blood glucose level measured by the BGM 106 or computed using data received from the CGM 102 is used to identify a progression or regression in the diabetic condition of the user 100. The blood glucose level measured by the BGM 106 or computed using data received from the CGM 102 is used to adjust (e.g., optimize) a therapy associated with the user 100.

In some embodiments, the user 100 uses an ambulatory non-durable insulin pump 116 or an ambulatory durable insulin pump 118 to treat the diabetic condition with insulin. The mobile device 104 determines an amount of insulin to be administered to the user 100 and the insulin pump 116, 118 receives instructions from the mobile device 104 to deliver a predetermined amount of insulin to the user 100. The insulin pump 116, 118 receives other information from the mobile device 104, such as mealtime information or exercise information of the user 100. The insulin pump 116, 118 determines the amount of insulin to administer based on the received information from the mobile device 104. The insulin pump 116, 118 communicates information to the mobile device 104. The information communicated to the mobile device 104 includes an amount of insulin delivered to the user 100, corresponding times of delivery, or a pump status (*e.g*., battery status, insulin status, or another status of a portion of the pump). The blood glucose level measured by the BGM 106 or computed using data received from the CGM 102 is used to adjust a configuration of the insulin pump 116, 118 or reduce a supply of insulin to the user in response to a progression or regression of the diabetic condition.

In some embodiments, the user 100 wears a fitness tracker 114. The fitness tracker 114 is a fitness band (*e.g.,* as shown in FIG. 1), a smart watch, or another wearable device. The fitness tracker 114 measures and accounts for the user's activity or lack thereof. The fitness tracker 114 is used to measure activity, such as walking, motion, running, sleeping, being inactive, bicycling, exercising on an elliptical trainer, and the like. The data (*e.g.,* activity data) collected by the fitness tracker 114 is transferred to and viewable on a computing device (*e.g*., such as the mobile device 104).

In some embodiments, the user 100 receives pre-packaged meals 130 to consume during the testing period. Each of the pre-packaged meals 130 includes a meal machine readable optical label 131. The meal machine readable optical label 131 is a bar code, a QR code, or some other unique identifier. The user 100 scans the meal machine readable optical label 131 prior to consuming the meal, for example, to confirm that the respective pre-packaged meal 130 was consumed.

In some embodiments, the user 100 receives pre-packaged medication doses 132 to take during the testing period. Each of the pre-packaged medication doses 132 includes a medication machine readable optical label 133. The medication machine readable optical label 133 is a bar code, a QR code, or some other unique identifier. The user 100 scans the medication machine readable optical label 133 prior to taking the medication dose, for example, to confirm that the respective pre-packaged medication dose 132 was taken.

In some embodiments, the user 100 uses a smart plate 128 to consume meals. The smart plate 128 includes one or more sensors configured to determine how much of the meal *(e.g.,* the pre-packaged meal) that the user 100 has eaten. The smart plate 128 is configured to scan the meal machine readable optical label 131 of a pre-packaged meal 130. For example, the smart plate 128 includes a camera or barcode scanner.

The mobile device 104 communicates with the insulin pump 116, 118, the CGM 102, the BGM 106, the fitness tracker 114, and the smart plate 128 using wired or wireless communications. The mobile device 104, the CGM 102, a CGM controller, the BGM 106, the fitness tracker 114, the smart plate 128, and the insulin pump 116, 118 are collectively referred to as user devices. The mobile device 104 communicates with the insulin pump 116, 118, the CGM 102, the BGM 106, the fitness tracker 114, and the smart plate 128 using the same or different wireless protocols. For example, the mobile device 104 communicates with the insulin pump 116, 118, the CGM 102, the BGM 106, the fitness tracker 114, and the smart plate 128 using BLUETOOTH^{®}, near field communication (NFC), THREAD^{®}, WIFI^{®}, ZIGBEE^{®}, WI-MAX^{®}, a cellular communication protocol, a proprietary wireless communication protocol, or another radio frequency (RF) communication protocol.

The mobile device 104 receives data and stores data for assisting in monitoring or treating the diabetic condition. The mobile device 104 receives input from the user 100 via a user interface (*e.g.,* a graphical user interface (GUI)) being provided on a display of the mobile device 104. The mobile device 104 receives input via hard buttons or soft buttons provided on the display. The user 100 confirms compliance with one or more aspects of the structured testing period via the user interface of the mobile device. For example, the user 100 confirms, via the mobile device 104, whether meals were consumed, medication doses were taken, and activities were participated in.

The mobile device 104 is configured to determine the device's location. For example, the mobile device 104 is able to determine the geolocation (*e.g*., latitude and longitude) of the device using signals from a global positioning system (GPS) or triangulation via cellular communications. The mobile device 104 determines a relative location using an RF beacon device 126. The RF beacon device 126 communicates a unique identifier via a short-range wireless communication, such as a BLUETOOTH^{®} low energy (BLE) beacon or an NFC beacon. The mobile device 104 receives the RF beacon and perform a lookup in a database (*e.g*., in information from the datastores 124) to determine a relative location associated with the unique identifier. For example, the mobile device 104 determines that the RF beacon indicates that the device is in a particular room in a home or building, on a certain floor in a building, close to a predefined object, or is within the RF range of a beacon associated with another object or location.

Some embodiments of the mobile device 104 include one or more sensors for detecting a relative position of the device or information about the user 100. The mobile device 104 detects a movement or a change in orientation. Based on the movement or change in orientation (or lack thereof) of the mobile device 104 over a period of time, the mobile device 104 detects that the user 100 is standing, sitting, or lying down. The mobile device 104 detects that the user 100 is exercising when the movement or a change in orientation is greater than a threshold for a period of time. The mobile device 104 detects the heartrate of the user 100 using a heartrate sensor. Based on the heartrate and the movement of the user 100 over a period of time, the mobile device 104 detects whether the user 100 is asleep or awake. The information about the mobile device 104 or the user 100 is used to provide information about or treat the diabetic condition.

The mobile device 104 provides information to the user 100 about the user's diabetic condition. For example, the mobile device 104 provides blood glucose levels, provides meal-related information, provides exercise-related information, generates graphs and other graphical user interfaces for display, or generates alerts that are provided to the user 100. For example, the mobile device 104 measures the blood glucose level of the user 100 and provides an alert when the blood glucose level of the user 100 has reached a threshold for an extreme diabetic state (*e.g.,* hypoglycemia or hyperglycemia). The alerts provided by the mobile device 104 are audible or non-audible alerts. The non-audible alerts are provided as a vibration, a flashing of the screen, or a flashing of an LED on the mobile device 104. The alerts also, or alternatively, are provided by an external device based on a communication from the mobile device 104. Some embodiments of the mobile device 104 include an electric motor for providing on-body vibration alerts or a speaker for providing audible alerts in response to data indications or triggers identified in the monitored blood glucose data.

The mobile device 104 communicates with other devices directly via a wired communication or a short-range wireless communication (*e.g*., WI-FI^{®}, BLUETOOTH^{®}, BLE, NFC, or another suitable short-range wireless communication). The mobile device 104 communicates indirectly with remote computing devices 122 or datastores 124 via a network 120 (*e.g*., using a WI-FI^{®} network, a cellular network, a WI-MAX^{®} network, or another wired or wireless network). The network 120 is a wired or wireless network. The network 120 is used to communicate over the Internet to other devices. One or more of the remote computing devices 122 are configured to execute a remote service application 123.

The mobile device 104 communicates with the remote computing devices to generate user interfaces for display on the mobile device 104, perform remote computation, or to otherwise control a remote computing device. For example, the mobile device 104 provides a user interface via an application or web browser that is generated at a remote computing device 122. The mobile device 104 generates instructions for providing reminders based on information associated with the testing period. The mobile device 104 displays one or more reminders to the user 100. The reminders are configured to remind the user 100 to input data, take medication, consume a meal, participate in an activity, and take a blood glucose measurement.

The mobile device 104 communicates with the datastores 124 to store information or retrieve information. The information includes information related to the user 100, the CGM 102, the BGM 106, the fitness tracker 114, the smart plate 128, and the insulin pump 116, 118. For example, the mobile device 104 receives treatment information associated with the user 100 as input or receive blood glucose information from the CGM 102 or the BGM 106 and send the information to the datastores 124 via the network 120. As another example, the mobile device 104 receives activity information from the fitness tracker 114 and sends the activity information to the datastores 124 via the network 120. Stored information is retrieved from the datastore 124 for treatment of the diabetic condition of the user. For example, the mobile device 104 retrieves an amount of insulin delivered to the user 100 or corresponding times of delivery. The datastores 124 include one or more remote storage locations, which are collectively referred to as cloud storage. For example, the datastores 124 store information regarding one or more personal characteristics of the user 100 (*e.g.,* the user's age or gender) or one or more alert profiles for an alert.

One or more of the user devices are implemented for reliable capture of testing period data from a user (*e.g*., during a structured period of testing). The testing period data includes blood glucose data, dietary intake data, medication data, and activity data. For example, the mobile device 104 receives testing period data (*e.g.,* blood glucose data, dietary intake data, medication data, and activity data) during the structured period of testing. The mobile device 104 executes a mobile application 105 for collecting the testing period data. The mobile device 104 (*e.g.,* via the mobile application 105) is configured to collect the testing period data, setup testing protocols, receive reminders, and enable user input of data. The mobile device 104 collects the testing period data via communication with one or more of the user devices. For example, the one or more user devices send testing period data to the mobile device 104. The mobile device 104 receives user input of data. For example, the mobile application 105 provides the user interface for the user 100 to input testing period data.

A length of the structured period of testing (*e.g.*, testing period) is determined based on the user and their disease status. In examples, the testing period is two or more days (*e.g*., three days) long. In other examples, the testing period is one day. The length of the testing period is adjusted during the testing period. For example, the testing period is extended (*e.g*., by one or more days) when testing period data is not received by the mobile device 104. The testing period data collection frequency is variable on different days of the testing period. For example, testing period data is collected more frequently on the first day of the testing period than the second or third day of the testing period.

The user 100 sets up one or more testing protocols using the mobile application 105. The testing protocols include the types of testing period data to collect and the frequency with which the testing period data will be collected. The mobile device 104 (*e.g.,* via the mobile application 105) sends one or more reminders to the user. The reminders are configured to remind the user to input data, take medication, consume a meal, participate in an activity, or take a blood glucose measurement.

Ideally, all of the testing period data would be collected and reported (*e.g.,* to the mobile device 104). During the testing period, one or more portions of data may be missed (*e.g.*, not received by the mobile device 104). For example, the mobile device 104 determines that one or more portions of data (*e.g.,* blood glucose data, dietary intake data, medication data, and activity data) was not received. The mobile device 104 or the remote computing device(s) 122 analyzes the testing period data to determine which type of data is missing. For example, the missing portion(s) of data is/are not received by the mobile device 104 due to one or more of the user forgetting to input the data (*e.g.,* into a mobile application 105), the mobile device 104 being powered off, or the wireless communications of the mobile device 104 being turned off. A complete set of data is needed from the structured testing period; so, when data is missing the testing period is extended (*e.g.,* a day is added to the testing period). Extending the testing period provides another opportunity for a complete set of data to be collected. But, extending the testing period is intrusive for the user and requires more processing resources on the mobile device 104 and other user devices. Continued testing drains the battery of the mobile device 104 due to the use of more processing resources to receive additional testing period data and update the learning model additional times.

Imputation of data on each day of the testing period is limited, for example, to ensure quality of the testing period data. In examples, one imputation is allowed on each day of the testing period. In examples, two imputations are allowed on day two or later of the testing period. More imputations may be allowed on later days of the testing period because the learning model used to predict the missing data is more accurate on the later days of the testing period. If more portions of data are missing on a day of the testing period than the allowable number of imputations, the testing period is extended even if the data is otherwise imputable with substitute data.

To collect the testing period data using the mobile application 105 (*e.g.,* a data collection application) on the mobile device 104 for monitoring the testing period data, the mobile device 104 uses unnecessary resources when the testing period is extended and the testing period data is communicated with the remote computing devices 122 (*e.g.,* remote software application 123) due to the inaccuracy of the data being obtained and the necessity to have to collect testing period data on additional testing days of the testing period. Extending the testing period increases the number of communications that are performed between the mobile device 104 and other devices (*e.g*., such as the remote computing devices 122, the fitness tracker 114, and the meal plate 128) which create additional traffic on wireless communication networks.

In some embodiments, the mobile device 104 or the remote computing device(s) 122 performs retroactive data imputation. The mobile device 104 (*e.g.,* via the mobile application 105) or the remote computing device(s) 122 (*e.g.,* via the remote service application 123) imputes the missing portion of data using contextual information, for example, after the determination that the portion of testing period data is missing. The missing portion of data is imputed with substitute data. Contextual information includes blood glucose data. For example, the contextual information includes an onset time, an acting time, and a peak associated with the blood glucose data. The contextual information used for imputation of the missing data is proximate to a pre-scheduled event or an unscheduled event. The substitute data includes confirmation that a pre-scheduled event occurred, blood glucose data, or confirmation that an unscheduled event occurred. The mobile device 104 imputes missing data at the end of each day of the testing period. In examples, the mobile device 104 notifies the user that data was missing and that the missing data was imputed with substitute data. As an option, the mobile device 104 requests that the user confirm the accuracy of the imputed substitute data.

When the missing portion of data corresponds to a pre-scheduled event, the mobile device 104 (*e.g.,* via the mobile application 105) uses blood glucose data proximate to the time that the pre-scheduled event was scheduled to take place. For example, the mobile device 104 analyzes the blood glucose data to determine whether the pre-scheduled event occurred. The analyzed blood glucose data includes onset time, acting time, and peak. When the mobile device 104 determines that the pre-scheduled event occurred, the mobile device 104 imputes the missing portion of data with substitute data. In this case, the substitute data includes a confirmation that the pre-scheduled event occurred.

When the mobile device 104 (*e.g.,* the mobile application 105) identifies a change in blood glucose data that is not proximate to a pre-scheduled event, the mobile device determines whether the change in blood glucose data corresponds to an unscheduled event. The mobile device 104 then determines which type of unscheduled event to which the change in blood glucose data corresponds, *e.g.*, based on analysis of the blood glucose data. When the mobile device 104 determines which type of unscheduled event the change in blood glucose data corresponds, the mobile device 104 imputes the missing portion of data with substitute data (*e.g*., indicates that the unscheduled event occurred).

The mobile device 104 calculates a confidence level for an imputation (*e.g*., each instance of imputation). The confidence level is calculated based on an analysis of the contextual information. For example, the mobile devices 104 assigns different weights to onset time, acting time, and peak for the imputation. The mobile device 104 aggregates the weights to calculate the confidence level associated with the imputation. The mobile device 104 increases the calculated confidence level for the imputation when the user confirms the accuracy of the imputed data.

In examples, the mobile device 104 increases the frequency, volume, or vibration intensity of reminders and notifications for the remainder of the day of the testing period, in response to an instance of imputation,
The mobile device 104 determines whether to extend the testing period based on the confidence level associated with the imputation. If the confidence level is greater than a pre-defined confidence threshold, the mobile device 104 determines not to extend the testing period. If the confidence level is less than or equal to the pre-defined confidence threshold, the mobile device 104 determines to extend the testing period. Extending the testing period includes adding one or more days to the testing period.

The mobile device 104 determines a daily aggregate confidence level by aggregating the confidence levels for each instance of substitute data imputation on a day of the testing period. The mobile device 104 extends the testing period in response to the daily aggregate confidence level being below the pre-defined confidence threshold.

The mobile device 104 determines whether to extend the testing period based on a number of imputations. If the number of imputations on a day of the testing period is too high, the mobile device 104 determines to extend the testing period. The number of imputations being too high depends on the day of the testing period. In examples, two or more imputations on the first day of the testing period is too high. In examples, three or more imputations on the second or later day of the testing period is too high. In examples, if the number of imputations in the overall testing period is too high, the data is unusable and another testing period is required.

Imputation of missing data is not based off of imputed data from a prior day of the testing period. At the end of each day of the testing period, the mobile device 104 (*e.g.,* using the mobile application 105) reports to the user which data was imputed, for example, as an educational checkpoint to increase compliance. In examples, the mobile device 104 (*e.g.,* using the mobile application 105) provides one or more tips for behavioral change(s) to increase compliance.

The mobile device 104 (*e.g.,* using the mobile application 105) or one or more of the remote computing devices 122 (*e.g.,* using the remote service application 123) analyzes disease progression and optimizes therapy based on the testing period data. For example, the testing period data enables the remote computing device 122 to identify a more appropriate (*e.g*., optimal) medication for the user 100. When a medication is selected for a user 100 using incomplete or inaccurate testing data, a suboptimal medication is selected for the user 100 which could result in a faster progression of the user's disease. Whereas when a medication is selected for the user 100 using complete data (*e.g*., with data imputation), progression of the user's disease can be slowed or the need for the user 100 to take insulin can be delayed.

In some embodiments, the mobile device 104 performs proactive data imputation. Proactive data imputation reduces processing resources required of the mobile device 104 during the testing period and reduces wireless communications bandwidth by reducing the instances of testing period data reports. In examples, a frequency of blood glucose information reports/receipt is adjustable during the testing period. Frequent reports of blood glucose information (*e.g*., from the CGM 102 to the mobile device 104) consumes device resources (*e.*g., processing capacity, battery power, or other device resources). Less frequent reporting of blood glucose information saves device resources and reduces wireless communications bandwidth. For example, the mobile device 104 receives blood glucose information (*e.g*., from the CGM 102) at a first frequency during the first day of the testing period and at a second frequency during the second day of the testing period. The second frequency is less than the first frequency such that the time interval between blood glucose information reports is greater on the second day of the testing period. The mobile device 104 imputes (*e.g*., by interpolation based on the blood glucose information received on the first day of the testing period) blood glucose data between reports on the second day of the testing period such that the amount of blood glucose information is substantially the same.

In some embodiments, the mobile device 104 performs predictive learning. For example, the mobile device 104 updates a learning model of the user 100 during the testing period. At the beginning of the testing period, the learning model includes data from a generic population-based model. The generic population-based model includes predictive blood glucose level responses using data from persons having a similar profile (*e.g.,* age, gender, weight, duration of disease, disease progression, or type of medication) to the user. At the end of the first day of the testing period, the learning model is updated to include predictive blood glucose level responses from the user 100 on the first day of the testing period. At the end of the second day of the testing period, the learning model is updated to include predictive blood glucose level responses from the user 100 on the second day of the testing period. The learning model is time-weighted such that blood glucose level responses of the user closest to the current day of the testing period are assigned a higher relative weight.

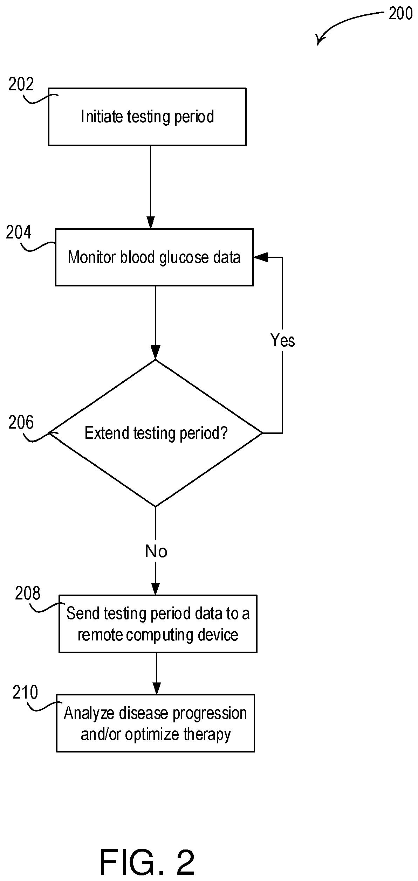

FIG. 2 shows a flowchart of an example process 200 for using a structured testing period to analyze disease progression and optimize therapy. The example process 200 is performed by one or more devices, such as one or more user devices, for example. The process 200 is performed by a single user device, or distributed across multiple devices. For example, the process 200 is performed by a computing device (e.g., such as mobile device 104 or remote computing device(s) 122 shown in FIG. 1). Portions of the process 200 are performed by other devices, such as a blood glucose monitor (*e.g*., the CGM 102 or the BGM 106 shown in FIG. 1).

As illustrated in FIG. 2, at 202 a testing period is initiated. The testing period is an infrequent, defined period of data capture. For example, the testing period is two or more days (*e.g.,* three days). The testing protocol may require information multiple times a day over the course of the testing period. For example, the testing protocol may require information three times a day over the course of the testing period.

At 204, the mobile device or the blood glucose monitor monitors blood glucose data during the testing period. In examples, the mobile device receives the blood glucose data from the blood glucose monitor. The blood glucose data is received at regular intervals during the testing period. In examples, the intervals are different on the first day, the second day, and the third day of the testing period. The blood glucose data includes a blood glucose level at a specific time. For example, the blood glucose level of the user is measured before and after a meal, before and after taking a medication, before and after an activity, and before and after bedtime. The blood glucose data includes a fasting blood glucose level. For example, the blood glucose level of the user is measured before bedtime, overnight (*e.g*., between midnight and 6am), and when the user wakes up.

At 206, the mobile device determines whether to extend the testing period. The mobile device determines to extend the testing period when more than one instance of blood glucose data is missing. The mobile device determines to extend the testing period when one instance of blood glucose data is missing and the missing data cannot be imputed with substitute data. When the mobile device determines to extend the testing period, the mobile device adds one or more days to the testing period and continue to monitor blood glucose data, at 204, on the added day(s). After the added day(s), the mobile device again determines whether to extend the testing period. When the mobile device determines to not extend the testing period, the mobile device sends, at 208, the testing period data to the remote service application operating on the remote computing devices that may be operated by a health care professional (HCP). The mobile device sends, at 208, the testing period data at the end of each day of the testing period or at the end of the testing period.

At 210, the remote service application at the remote computing device or the mobile device analyzes the testing period data to determine disease progression and optimize therapy. For example, the testing period data enables identification of a progression or a regression in a diabetic condition associated with the user. In examples, one or more configurations of an insulin pump (*e.g.,* such as the ambulatory non-durable insulin pump 116 or the ambulatory durable insulin pump 118 shown in FIG. 1) is adjusted to increase or reduce the supply of insulin to the user in response to the progression or the regression in the diabetic condition. In examples, an amount of medication or a frequency of medication is adjusted in response to the testing period data.

The remote computing device or the mobile device compares the testing period data to previous testing period data from a previous testing period of the user. In other examples, the remote computing device or the mobile device compares the testing period data to a generic population model. The remote computing device or the mobile device establishes a baseline disease progression of the user, for example, if the testing period is the first testing period of the user. The remote computing device updates a previous user-specific model (*e.g.,* a previous learning model), for example, if the testing period is a second or later testing period of the user.

Each day of a testing period includes an analysis of received testing period data to determine whether any data is missing. When a portion of the testing period data is missing, the mobile device determines whether the missing data can be imputed with substitute data. If the missing data can be imputed with substituted data, a confidence level of the imputed substitute data is calculated. When the confidence level of the imputed substitute data is above a pre-defined threshold (*e.g*., 90%), the substitute data is added to the testing period data for the day. The testing period data for the day is used to update a learning model associated with the user.

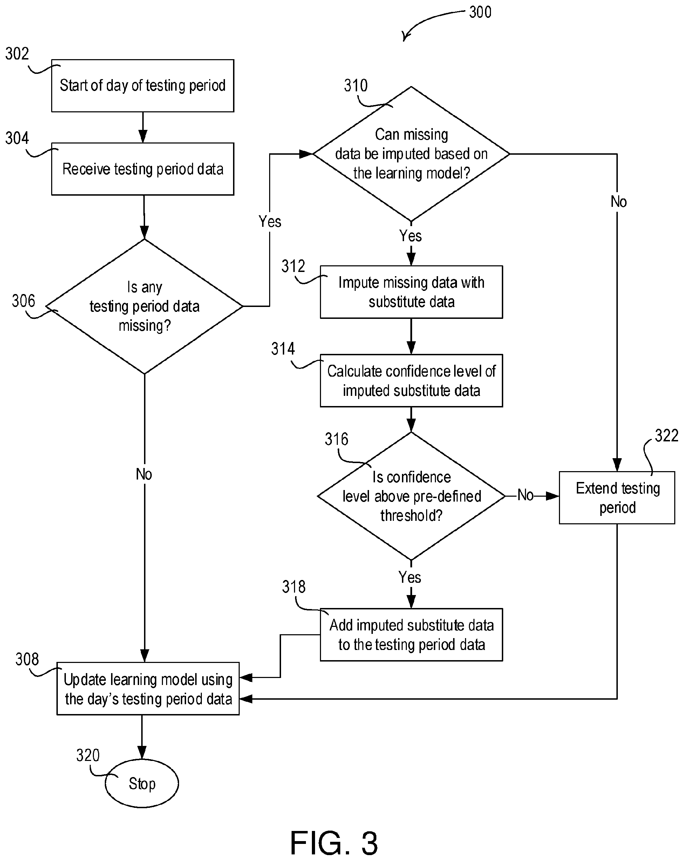

FIG. 3 shows a flowchart of an example process 300 for a day of a structured testing period. The example process 300 is performed by one or more devices, such as one or more user devices, for example. The process 300 is performed by a single user device, or distributed across multiple devices. For example, the process 300 is performed by a computing device (*e.g*., such as mobile device 104 shown in FIG. 1). The process 300 is performed on each day of the testing period. The testing period is an infrequent, defined period of data capture. For example, the testing period is two or more days (*e.g*., three days). For example, the process 300 is initiated at the start of each day of the testing period.

As illustrated in FIG. 3, at 302 a day of the testing period starts. The mobile device indicates (*e.g*., via display, reminder, or alert) to the user that the day of the testing period has started. The day of the testing period starts when the user wakes up. For example, the day of the testing period starts when a morning fasting blood glucose level of the user is received by the mobile device.

At 304, the mobile device receives testing period data during the testing period. The testing period data includes blood glucose data, dietary intake data, activity data, and medication data. The mobile device receives blood glucose data from a blood glucose monitor (*e.g*., the CGM 102 or the BGM 106 shown in FIG. 1). The blood glucose data includes one or more blood glucose levels of the user that are measure during the testing period. The blood glucose data is received at regular intervals during the testing period. In examples, the intervals are different on the first day, the second day, or the third day of the testing period. The blood glucose data includes a blood glucose level (*e.g*., measurement) at a specific time. For example, the blood glucose level of the user is measured before and after a meal, before and after taking a medication, before and after an activity, and before and after bedtime. The blood glucose data includes a fasting blood glucose level. For example, the blood glucose level of the user is measured before bedtime, at midnight, and when the user wakes up.

The dietary data includes whether the user consumed a meal, a carbohydrate content of the meal, nutrition information associated with the meal (*e.g.,* a glycemic profile of the meal, macronutrient information of the meal, a carbohydrate content of the meal), a pre-determined meal blood glucose level increase, a percentage of the meal that the user consumed, or macronutrient information of the meal. The activity data includes whether user participated in the activity, a type of the activity (*e.g*., pre-scheduled activity), a length of the activity, or a pre-determined activity blood glucose level increase. The medication data includes whether the user took a medication dose, a type of medication, a dynamic fingerprint associated with the medication, or a pre-determined medication blood glucose level increase.

The testing period data is received via wireless communications via the mobile application 105 executed on the mobile device. For example, the mobile application 105 is pre-loaded with one or more of scheduled meals, scheduled medications, or scheduled activities. The mobile application 105 enables the user to indicate whether the scheduled meals were eaten, whether the scheduled medications were taken, and whether the scheduled activities were performed.

In examples, dietary intake data is input by the user into the mobile application 105. For example, the user indicates via the mobile application 105 whether a meal was consumed and at what time the meal was consumed. The meal is a large meal, a small meal, or a snack. The user inputs into the mobile application 105 the nutrition information associated with the meal and how much of the meal was consumed. Additionally or alternatively, the meals are pre-packaged meals (*e.g.,* such as the pre-packaged meals 130 shown in FIG. 1). Each of the pre-packaged meals includes a machine readable optical label (*e.g.,* such as the meal optical label 131 shown in FIG. 1) that is scannable to determine nutrition information of the pre-packaged meal. The machine readable optical label indicates the nutrition information. For example, a device (*e.g.,* the mobile device) retrieves (*e.g*., from memory, from the internet, or a database) the nutrition information for the meal based on the machine readable optical label.

In examples, the user consumes the meal using a smart plate (*e.g.,* such as the smart plate 128 shown in FIG. 1). The smart plate includes one or more sensors configured to determine how much of the meal (*e.g.,* the pre-packaged meal) that the user has eaten. The smart plate is configured to scan the machine readable optical label of the pre-packaged meal. For example, the smart plate includes a camera or barcode scanner.

In examples, activity data is input by the user into the mobile application 105. For example, the user indicates via the mobile application 105 whether the user participated in an activity and at what time of day. The activity is a pre-scheduled activity or an unscheduled activity. Examples of activities include running, jogging, walking, yoga, strength training, interval training, and other exercise types. The user also inputs a length of the activity into the mobile application 105. Additionally or alternatively, the mobile device receives data from a fitness tracker (*e.g*., such as the fitness tracker 114 shown in FIG. 1) worn by the user. The fitness tracker is a fitness band, a watch, or some other wearable device that is equipped with one or more sensors that are configured to track activity levels during the testing period. For example, the data from the fitness tracker indicates when the user participates in a pre-scheduled activity or an unscheduled activity.

In examples, medication data is input by the user into a mobile application (*e.g*., the mobile application 105 shown in FIG. 1). For example, the user indicates via the mobile application whether the user took a medication dose. The medication dose is a pre-scheduled medication dose or an unscheduled medication dose. Additionally or alternatively, the medication doses are pre-packaged medication doses (*e.g*., such as the medication doses 132 shown in FIG. 1). Each of the pre-packaged medication doses includes a machine readable optical label (*e.g.,* such as the medication optical label 133 shown in FIG. 1) that is scannable to indicate that the user took the medication dose. The machine readable optical label indicates the nutrition information. For example, a device (*e.g.,* the mobile device) retrieves (*e.g.,* from memory, from the internet, or from a database) the nutrition information for the meal based on the machine readable optical label.

At 306, the mobile device determines whether any testing period data is missing. The mobile device determines whether dietary data, activity data, medication data, or blood glucose data is missing based on analysis of the received testing period data.

When the mobile device determines that no testing period data is missing from the day of the testing period, the mobile device updates, at 308, a learning model associated with the user (*e.g.,* the time-weighted blood glucose model) using the day's testing period data. The updated learning model is used on future days of the testing period to impute testing period data or identify whether missing data can be imputed by substitute data. The process 300 stops at 320.

When the mobile device determines that a portion of testing period data is missing, the mobile device determines, at 310, if the missing portion of the testing period data can be imputed, for example, based on analysis of the learning model and the blood glucose data. The learning model is populated with generic population based data on a first day of the testing period. For example, on the first day of the testing period, the learning model includes predictive blood glucose level responses using data from persons having a similar profile (*e.g.,* age, gender, weight, duration of disease, disease progression, or type of medication) to the user.

The mobile device determines, at 310, which type of data is missing. For example, the mobile device analyzes one or more scheduled meals, one or more scheduled medication doses, or one or more pre-scheduled activities to determine which type of data is missing. The mobile device determines that the missing portion of the testing period data comprises dietary intake data based on an analysis of one or more scheduled meals. The mobile device determines that the missing portion of data comprises medication data based on an analysis of one or more scheduled medication doses. The mobile device determines that the missing portion of the testing period data comprises activity data based on an analysis of one or more pre-scheduled activities.

The learning model is populated with time-weighted testing period data of the user on the second, third, and subsequent days of the testing period. For example, the learning model includes predictive blood glucose level responses using testing period data from previous days of the testing period. For example, the mobile device compares one or more measured blood glucose levels proximate to the missing portion of the testing period data to one or more blood glucose levels in the learning model. The mobile device determines based on the analysis of the learning model whether the user consumed a meal associated with the missing portion of testing period data, whether the user participated in an activity associated with the missing portion of testing period data, or whether the user took a medication dose associated with the missing portion of testing period data. In examples, the mobile device determines whether the missing portion of testing period data corresponds to blood glucose data, dietary intake data, medication data, or activity data.

When the mobile device determines that the missing portion of data cannot be imputed, the mobile device extends, at 322, the testing period. The mobile device determines that the missing portion of data cannot be imputed when analysis of the learning model is inconclusive. In examples, the mobile device may be unable to determine from analysis of the learning model whether a meal was consumed, a medication dose was taken, or an activity was participated in. When the testing period is extended, the mobile device adds an additional day to the testing period. When the testing period is extended, the mobile device updates, at 308, the learning model using testing period data received on that day of the testing period.

When the mobile device determines that the missing portion of data can be imputed, the mobile device imputes, at 312, the missing portion of data with substitute data. The mobile device uses contextual information to determine the substitute data. In examples, the mobile device uses the learning model to impute the missing portion of data.

At 314, the mobile device calculates a confidence level of the imputed substitute data. The confidence level of the imputed substitute data is dependent on the type of data that is missing. For example, substitute data imputing a missing before-meal blood glucose level is assigned a higher confidence level than substitute data imputing a missing after-meal blood glucose level. Imputation of the before-meal blood glucose level is assigned a higher confidence level because the after-meal blood glucose level is more critical to updating the learning model, the disease progression analysis, and the therapy analysis.

At 316, the mobile device determines whether the confidence level of the imputed substitute data is greater than a pre-defined confidence threshold. The pre-defined confidence threshold is 90%. When the confidence level of the imputed substitute data is greater than the pre-defined confidence threshold, the mobile device displays a notification requesting the user confirm the imputed substitute data. When the confidence level of the imputed substitute data is greater than the pre-defined confidence threshold, the mobile device adds, at 318, the imputed substitute data to the testing period data. The testing period data is then used (e.g., with the imputed substitute data), at 308, to update the learning model associated with the user. The testing period data is also used to analyze disease progression and optimize therapy, for example, such as step 210 of process 200 shown in FIG. 2. The process 300 ends at 320 after the learning model is updated with the testing period data from the day.

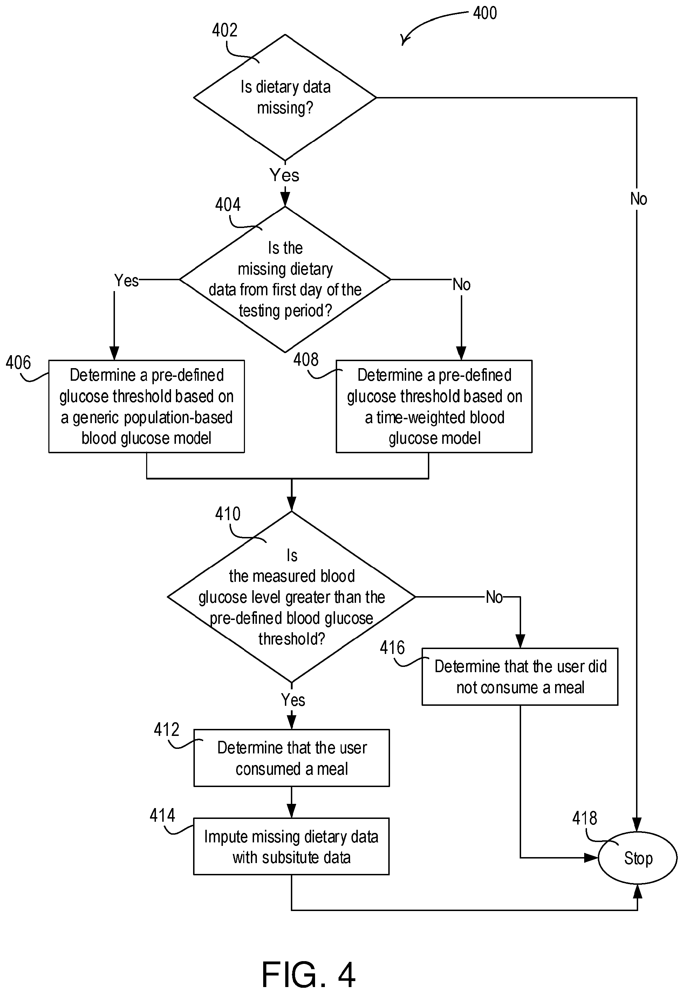

FIG. 4 shows a flowchart of an example process 400 for imputation of dietary data during a structured testing period. The example process 400 is performed by one or more devices, such as one or more user devices, for example. The process 400 is performed by a single user device, or distributed across multiple devices. For example, the process 400 is performed by a computing device (e.g., such as mobile device 104 shown in FIG. 1). The process 400 is initiated proximate to a time period that a meal is scheduled to be consumed by the user. Additionally or alternatively, the process 400 is initiated based on analysis of blood glucose data. For example, the process 400 is initiated by a blood glucose level increase. The process 400 is initiated at step 306 of the process 300 shown in FIG. 3.

As illustrated in FIG. 4, at 402 the mobile device determines whether dietary data is missing. During the testing period, the user inputs meal consumption parameters into the mobile device (*e.g.,* such as the mobile device 104 shown in FIG. 1) via a mobile application (*e.g.,* such as the mobile application 105 shown in FIG. 1). Meal consumption parameters include the food to be consumed, the amount of the foods to be consumed, and the nutrition information associated with the food. The mobile device determines that dietary data is missing when expected dietary data (*e.g*., meal consumption parameters) is not entered into the mobile application.

Before consuming the meal, the user scans a pre-packaged meal (*e.g.,* a bar code) to indicate which meal is about to be consumed. In examples, the pre-packaged meal includes a sensor in its packaging that communicates with the mobile device (*e.g*., via proximity-based communication, radio frequency identification (RFID), near-field communication (NFC), or BLUETOOTH^{®} communication protocol) to send details associated with the meal. The pre-packaged meal is associated with a serial number. The user inputs the serial number into the mobile application. The mobile application has the meal nutritional information associated with the meal stored in memory or retrieves the meal nutritional information from a food database (*e.g*., via the Internet). Additionally or alternatively, the user inputs nutrition information associated with the meal. For example, the user inputs the separate components of the meal and the mobile application populates the nutrition information. In this case, the mobile application maintains or accesses a food database to populate nutrition information. In examples, the user searches for and select the food items they will consume during each meal via a user interface that accesses or interfaces with the food database.

After consuming the meal, the user confirms what percentage of the meal was consumed. For example, the user confirms that the entire meal was consumed or that a portion of the meal was consumed. The mobile device initiates a meal consumption reminder. The mobile device initiates the meal consumption reminder a pre-determined amount of time after the meal consumption parameters were input. In examples, the user consumes the meal using a smart plate (*e.g.,* such as the smart plate 128 shown in FIG. 1). The smart plate includes one or more sensors configured to determine how much of the meal (*e.g.,* the pre-packaged meal) that the user has eaten. For example, the smart plate weighs the meal before and after the user has finished eating. In examples, the smart plate is configured to scan the machine readable optical label of the pre-packaged meal. For example, the smart plate includes a camera or barcode scanner. The smart plate sends nutrition information to the mobile device. For example, the smart plate indicates to the mobile device how much of the meal was consumed. When the mobile device determines that dietary data is not missing, the process 400 stops at 418.

When the mobile device determines that dietary data is missing, the mobile device determines, at 404, whether the dietary data is missing on the first day of the testing period. If the dietary data is missing on a first day of the testing period, the mobile device determines, at 406, a pre-defined blood glucose threshold for the missing dietary data based on a generic population-based blood glucose model. The generic population-based model approximates blood glucose level responses for a generic person with diabetes using one or more data sets from other people with diabetes of a similar age, the same gender, a similar duration of disease, or taking a similar medication.

If the dietary data is missing on a second, third, or later day of the testing period, the mobile device determines, at 408, the pre-defined blood glucose threshold based on a time-weighted blood glucose model associated with the user. The time-weighted blood glucose model is developed for the user using blood glucose data of the user received on one or more previous days (*e.g.,* the first day) of the testing period. For example, the time-weighted blood glucose model uses blood glucose level responses of the user in response to consuming a meal, taking a medication, and participating in an activity on the first day of the testing period. The time-weighted blood glucose model is continuously updated during the testing period. The time-weighted blood glucose model assigns a greater weight to data received proximate to the missing data. For example, a weight assigned to a specific portion of data decreases as the testing period progresses. Additionally or alternatively, the time-weighted blood glucose model is updated at the end of each day of the testing period.

At 410, the mobile device determines if a measured blood glucose level of the user is greater than the pre-defined blood glucose threshold. The mobile device analyzes one or more blood glucose levels proximate to a time period associated with the meal. As an example, the time period includes 11am to 1pm when the meal is lunch. The mobile device compares a measured blood glucose level of the user proximate to a time associated with the meal (*e.g.,* after the meal). When the measured blood glucose level of the user proximate to the time associated with the meal is less than or equal to the pre-defined blood glucose threshold, the mobile device determines, at 416, that the user did not consume the meal. And, the process 400 stops at 418.

When the measured blood glucose level is greater than the pre-defined blood glucose threshold, the mobile device determines, at 412, that the user consumed the meal. The mobile device imputes, at 414, the missing dietary data with substitute data, for example, based on the determination that the user consumed the meal. The mobile device uses contextual information to determine the substitute data. Users often miscalculate an amount of food consumed or the nutritional information of the food consumed. Therefore, imputation of dietary intake data results in a more accurate data set because the imputed dietary intake data is likely more accurate than dietary intake data that is input by the user. The process 400 stops, at 418, when the missing dietary data is imputed with the substitute data.

Missing activity data can be imputed using a learning model associated with the user. The learning model is a generic population-based blood glucose model on a first day of the testing period. The learning model is a time-weighted blood glucose model on second and later days of the testing period. When a measured blood glucose level proximate to the missing activity data is greater than a pre-defined blood glucose threshold, it is determined that the user participated in the activity. The pre-defined blood glucose threshold is determined using the learning model. The missing activity data is imputed with substitute data when the user participated in the activity.

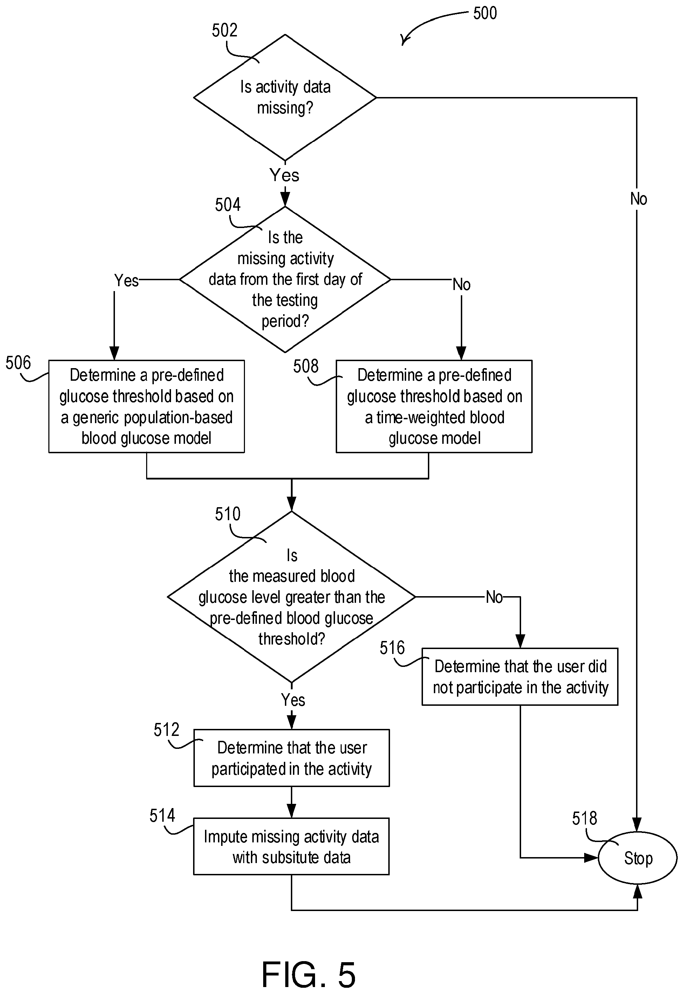

FIG. 5 shows a flowchart of an example process 500 for imputation of activity data during a structured testing period. The example process 500 is performed by one or more devices, such as one or more user devices, for example. The process 500 is performed by a single user device, or distributed across multiple devices. For example, the process 500 is performed by a computing device (*e.g*., such as mobile device 104 shown in FIG. 1). The process 500 is initiated proximate to a time period that an activity is scheduled to be participated in by the user. The activity is a pre-scheduled activity or an unscheduled activity. Additionally or alternatively, the process 500 is initiated based on analysis of blood glucose data. The mobile device receives blood glucose data (*e.g.,* from a blood glucose monitor) during the testing period. The blood glucose data includes one or more measured blood glucose levels of the user during the testing period. For example, the process 500 is initiated by a blood glucose level increase. The process 500 is initiated at step 306 of the process 300 shown in FIG. 3.

As illustrated in FIG. 5, at 502 the mobile device determines whether activity data is missing. During the testing period, the user inputs activity parameters into the mobile device (*e.g.,* such as the mobile device 104) via a mobile application (*e.g.*, such as the mobile application 105). Activity parameters include the activity type, a length of the activity, or a pre-determined activity blood glucose level increase associated with the user. The mobile device determines that activity data is missing when expected activity data (*e.g.*, activity parameters) is not entered into the mobile application. In examples, the mobile application receives the activity parameters from a fitness tracker worn by the user.

The mobile application has information associated with the activity stored in memory or retrieves the activity information from an activity database (*e.g*., via the Internet). The activity information includes an estimated level of effort or an estimated amount of calories expected to be burned during the activity. Additionally or alternatively, the user inputs the activity information. For example, the user inputs the type of activity and the length of the activity. The mobile application populates the activity information based on the type of activity and the length of the activity. In this case, the mobile application maintains or accesses an activity database to populate the activity information. In examples, the user searches for and selects the activity type they will participate in via a user interface that accesses or interfaces with the activity database.

When the mobile device determines that activity data is not missing, the process 500 stops at 518.

When the mobile device determines that activity data is missing, the mobile device determines, at 504, whether the activity data is missing on the first day of the testing period. If the activity data is missing on a first day of the testing period, the mobile device determines, at 506, a pre-defined blood glucose threshold for the missing activity data based on a generic population-based blood glucose model. The generic population-based model approximates blood glucose level responses for a generic person with diabetes using one or more data sets from other people with diabetes of a similar age, the same gender, a similar duration of disease, or taking a similar medication.

If the activity data is missing on a second, third, or later day of the testing period, the mobile device determines, at 508, the pre-defined blood glucose threshold based on a time-weighted blood glucose model associated with the user. The time-weighted blood glucose model is developed for the user using blood glucose data of the user received on one or more previous days *(e.g.,* the first day) of the testing period. For example, the time-weighted blood glucose model uses blood glucose level responses of the user in response to consuming a meal, taking a medication, and participating in an activity on the first day of the testing period. The time-weighted blood glucose model is continuously updated during the testing period. The time-weighted blood glucose model assigns a greater weight to data received proximate to the missing data. For example, a weight assigned to a specific portion of data decreases as the testing period progresses. Additionally or alternatively, the time-weighted blood glucose model is updated at the end of each day of the testing period.

At 510, the mobile device determines if a measured blood glucose level of the user is greater than the pre-defined blood glucose threshold. The mobile device analyzes one or more blood glucose levels proximate to a time period associated with the activity. As an example, the time period includes 6am to 9am when the activity is a morning jog. The mobile device compares a measured blood glucose level of the user proximate to a time associated with the activity (e.g., after the activity). When the measured blood glucose level is less than or equal to the pre-defined blood glucose threshold, the mobile device determines, at 516, that the user did not participate in the activity. And, the process 500 stops at 518.

When the measured blood glucose level is greater than the pre-defined blood glucose threshold, the mobile device determines, at 512, that the user participated in the activity. The mobile device determines, at 512, that the user participated in the activity based on an analysis of medication data proximate the time period associated with the activity. For example, if there are no medication doses proximate to the time period associated with the activity, the mobile device eliminates a dose of medication as a possible cause of an increased blood glucose level. The mobile device imputes, at 514, the missing activity data with substitute data, for example, based on the determination that the user participated in the activity. The mobile device uses contextual information to determine the substitute data. The process 500 stops, at 518, when the missing dietary data is imputed with the substitute data.

Missing medication data can be imputed using a learning model associated with the user. The learning model is a generic population-based blood glucose model on a first day of the testing period. The learning model is a time-weighted blood glucose model on second and later days of the testing period. When a measured blood glucose level proximate to the missing medication data is greater than a pre-defined blood glucose threshold, it is determined that the user took a medication dose. The pre-defined blood glucose threshold is determined using the learning model. The missing activity data is imputed with substitute data when the user took the medication dose.

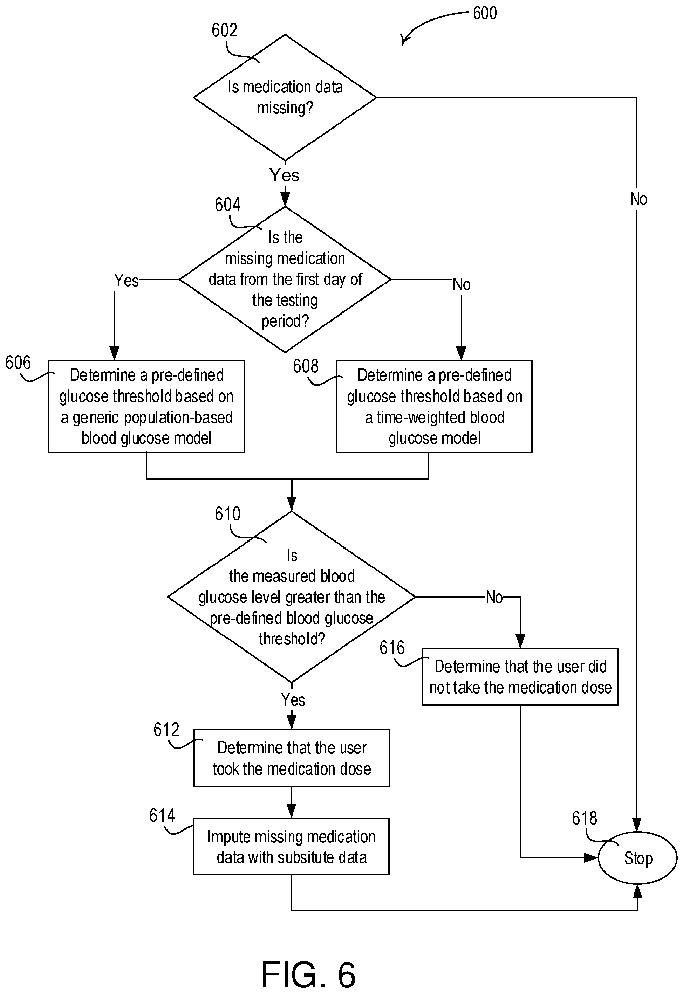

FIG. 6 shows a flowchart of an example process 600 for imputation of medication data during a structured testing period. The example process 600 is performed by one or more devices, such as one or more user devices, for example. The process 600 is performed by a single user device, or distributed across multiple devices. For example, the process 600 is performed by a computing device (e.g., such as mobile device 104 shown in FIG. 1). The process 600 is initiated proximate to a time period that a medication dose is scheduled to be taken in by the user. The medication dose is a pre-scheduled medication dose or an unscheduled medication dose. An unscheduled medication dose includes a pain reliever, an allergy medication, or a cold medication. The mobile device scans a bar code on the packaging of the unscheduled medication dose to input the medication data. Additionally or alternatively, the process 600 is initiated based on analysis of blood glucose data. The mobile device receives blood glucose data (e.g., from a blood glucose monitor) during the testing period. The blood glucose data includes one or more measured blood glucose levels of the user during the testing period. For example, the process 600 is initiated by a blood glucose level increase. The process 600 is initiated at step 306 of the process 300 shown in FIG. 3.

As illustrated in FIG. 6, at 602 the mobile device determines whether medication data is missing. During the testing period, the user inputs medication data (e.g., medication parameters) into the mobile device (e.g., such as the mobile device 104 shown in FIG. 1) via a mobile application (e.g., such as the mobile application 105 shown in FIG. 1). Additionally or alternatively, the mobile device is pre-populated with the medication data, for example, as part of an e-prescription process. The medication parameters include the medication type, a dosage amount of the medication, a time period associated with a dose of the medication, or a pre-determined medication blood glucose level increase associated with the user. The mobile device determines that medication data is missing when expected medication data (e.g., medication parameters) is not entered into the mobile application. The mobile application receives the medication parameters from a medical device (e.g., such as the ambulatory non-durable insulin pump 116 or the ambulatory durable insulin pump 118 shown in FIG. 1) that is configured to deliver a dose of medication.

The mobile application has information associated with the medication stored in memory or retrieves the medication information from a medication database (e.g., via the Internet). The medication information includes a dynamic fingerprint associated with a medicine of the medication dose, a kinetic fingerprint associated with the medicine of the medication dose, or a dosage amount of the medication. Additionally or alternatively, the user inputs the medication information. For example, the user inputs the type of type of medication and a dosage amount and the mobile application populates the medication information. In examples, the mobile application maintains or accesses a medication database to populate the medication information. The user searches for and selects the medication type they will take via a user interface that accesses the medication database. When the mobile device determines that medication data is not missing, the process 600 stops at 618.

When the mobile device determines that medication data is missing, the mobile device determines, at 604, whether the medication data is missing on the first day of the testing period. If the medication data is missing on a first day of the testing period, the mobile device determines, at 606, a pre-defined blood glucose threshold for the missing medication data based on a generic population-based blood glucose model. The generic population-based model approximates blood glucose level responses for a generic person with diabetes using one or more data sets from other people with diabetes of a similar age, the same gender, a similar duration of disease, or taking a similar medication. The pre-defined blood glucose threshold is determined based on the bodyweight of the user.

If the medication data is missing on a second, third, or later day of the testing period, the mobile device determines, at 608, the pre-defined blood glucose threshold based on a time-weighted blood glucose model associated with the user. The time-weighted blood glucose model is developed for the user using blood glucose data of the user received on one or more previous days *(e.g.,* the first day) of the testing period. For example, the time-weighted blood glucose model uses blood glucose level responses of the user in response to consuming a meal, taking a medication, and participating in an activity on the first day of the testing period. The time-weighted blood glucose model is continuously updated during the testing period. The time-weighted blood glucose model assigns a greater weight to data received proximate to the missing data. For example, a weight assigned to a specific portion of data decreases as the testing period progresses. Additionally or alternatively, the time-weighted blood glucose model is updated at the end of each day of the testing period.

At 610, the mobile device determines if a measured blood glucose level of the user is greater than the pre-defined blood glucose threshold. The mobile device analyzes one or more blood glucose levels proximate to a time period associated with the medication. As an example, the time period includes 7am to 9am when the medication dose is a multivitamin to be taken with breakfast. The mobile device compares a measured blood glucose level of the user proximate to a time associated with the medication dose *(e.g.,* after the medication dose). When the measured blood glucose level is less than or equal to the pre-defined blood glucose threshold, the mobile device determines, at 616, that the user did not take the medication dose. And, the process 600 stops at 618.

When the measured blood glucose level is greater than the pre-defined blood glucose threshold, the mobile device determines, at 612, that the user took the medication dose. The mobile device determines, at 612, that the user took the medication dose based on an analysis of activity data proximate the time period associated with the medication dose. For example, if there are no activities proximate to the time period associated with the medication dose, the mobile device eliminates an activity as a possible cause of an increased blood glucose level. The mobile device imputes, at 614, the missing medication data with substitute data, for example, based on the determination that the user took the medication dose. The mobile device uses contextual information to determine the substitute data. The process 600 stops, at 618, when the missing medication data is imputed with the substitute data.

The mobile device determines whether the user took a double dose of a medication, for example, to compensate for a missed dose. For example, the mobile device analyzes the measured blood glucose level to determine if the user took a double dose of the medication.

Missing blood glucose data is imputed. In examples, the missing blood glucose data is imputed using a learning model associated with the user. In examples, the missing blood glucose data is imputed using other blood glucose data. For example, one or more blood glucose levels proximate to the missing blood glucose data are estimated. The estimated blood glucose levels are used to impute the missing blood glucose data. A confidence level of the imputed blood glucose data is calculated.

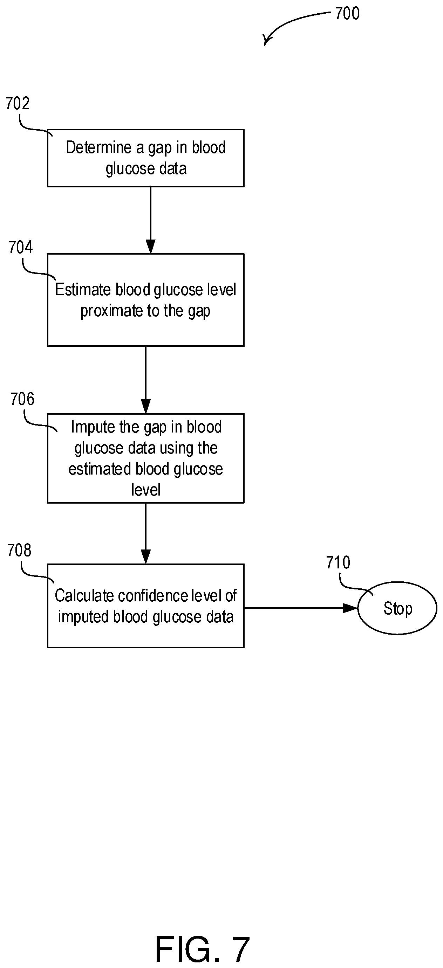

FIG. 7 shows a flowchart of an example process 700 for imputation of blood glucose data during a structured testing period. The example process 700 is performed by one or more devices, such as one or more user devices, for example. The process 700 is performed by a single user device, or distributed across multiple devices. For example, the process 700 is performed by a computing device (e.g., such as mobile device 104 shown in FIG. 1). The process 700 is initiated proximate to a time period associated with a meal, an activity, or a medication dose. Additionally or alternatively, the process 700 is initiated based on analysis of blood glucose data. The mobile device receives blood glucose data (e.g., from a blood glucose monitor) during the testing period. The blood glucose data includes one or more measured blood glucose levels of the user during the testing period. For example, the process 700 is initiated by a blood glucose level increase.

As illustrated in FIG. 7, at 702 the mobile device determines a gap in blood glucose data. For example, the mobile device receives blood glucose levels of the user at regular intervals during the testing period, before and after meals, before and after medication doses, and before and after activities. The mobile device determines, at 702, a gap in blood glucose data when one or more of the blood glucose levels is missing.

At 704, the mobile device estimates a blood glucose level of the user proximate to the gap. The mobile device uses contextual information to estimate the blood glucose level of the user. In examples, the mobile device estimates the blood glucose level of the user proximate to the gap by interpolating between received blood glucose levels. In examples, the mobile device estimates the blood glucose level of the user proximate to the gap using previously measured blood glucose levels associated with the same meal, activity, or medication dose.

At 706, the mobile device imputes the gap in blood glucose data with the estimated blood glucose level. At 708, the mobile device calculates a confidence level of the imputed blood glucose level. The confidence level is calculated based on one or more of the type of estimate, the day of testing period, or before/after estimate. For example, an interpolated blood glucose level estimate is assigned a higher confidence level than a previously measured blood glucose level estimate. An interpolated blood glucose level estimate on a later day (e.g., second or third day) of the testing period is assigned a higher confidence level than an interpolated blood glucose level estimate on an earlier day *(e.g.,* first day) of the testing period. An estimated blood glucose level before a meal, activity, or medication dose is assigned a higher confidence level than an estimated blood glucose level after a meal, activity, or medication dose. The process 700 stops, at 710, when the confidence level is calculated.

A fasting blood glucose level is imputed. A bedtime blood glucose level of the user is measured before the user goes to sleep. A morning fasting blood glucose level of the user is received when the user wakes up. If an overnight fasting blood glucose level of the user is not received, the overnight fasting blood glucose level is imputed with substitute data. In examples, the substitute data is an overnight fasting blood glucose level of the user on a different day of the testing period. In examples, the substitute data is determined by interpolating between the bedtime blood glucose level and the morning fasting blood glucose level.

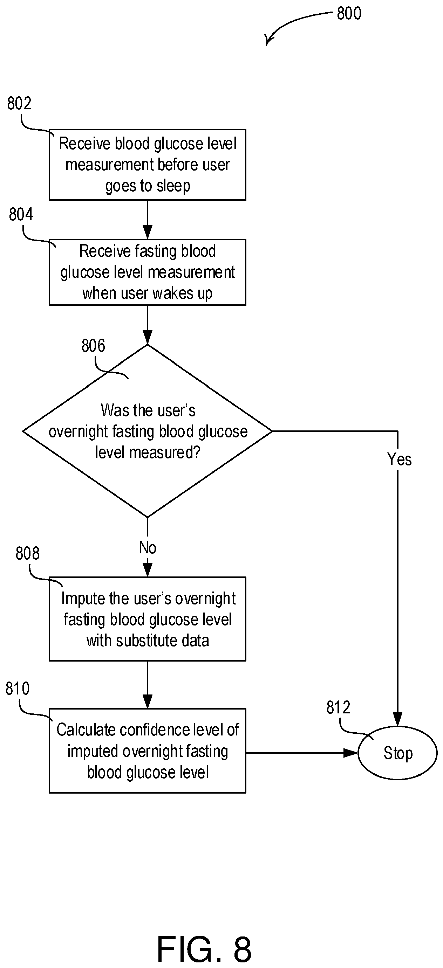

FIG. 8 shows a flowchart of an example process 800 for imputation of a fasting blood glucose level during a structured testing period. The example process 800 is performed by one or more devices, such as one or more user devices, for example. The process 800 is performed by a single user device, or distributed across multiple devices. For example, the process 800 is performed by a computing device (e.g., such as mobile device 104 shown in FIG. 1). The process 800 is initiated proximate to a bedtime of the user. For example, the mobile device initiates the process 800 at a pre-configured bedtime of the user. Additionally or alternatively, the user initiates the process 800 before going to sleep. The user initiates the process via a mobile application (*e.g*., such as the mobile application 105 shown in FIG. 1) running on the mobile device.

As illustrated in FIG. 8, at 802 the mobile device receives a blood glucose level measurement before the user goes to sleep. The blood glucose level measurement is received from a blood glucose monitor (*e.g*., such as the CGM 102 or the BGM 106 shown in FIG. 1). The blood glucose level measurement is stored in a memory of the mobile device.

At 804, the mobile device receives a blood glucose level measurement (*e.g*., a fasting blood glucose level) when the user wakes up. The mobile device determines (*e.g*., automatically) that the user is awake based on one or more of activity data, movement of the mobile device, or operation (*e.g*., unlocking) of the mobile device.

At 806, the mobile device determines whether the user's fasting blood glucose level was measured overnight (*e.g*., between midnight and 6:00 am). The overnight fasting blood glucose level is measured at a midpoint between bedtime and the time when the user wakes up. If the user's fasting blood glucose level of the user was measured overnight, the process 800 stops at 812. If the fasting blood glucose level of the user was not measured overnight, the mobile device imputes, at 808, the overnight fasting blood glucose level using substitute data. In examples, the mobile device determines the substitute data by interpolating between the blood glucose level measurement received before bedtime and the blood glucose level measurement received when the user woke up. In examples, the mobile device estimates the substitute data based on an overnight fasting blood glucose level measured on a previous day or a later day of the testing period.

At 808, the mobile device calculates a confidence level of the imputed overnight fasting blood glucose level. The confidence level is calculated based on the type of estimate or the day of testing period. For example, an interpolated overnight fasting blood glucose level estimate is assigned a higher confidence level than a previously or later measured overnight fasting blood glucose level estimate. An interpolated overnight fasting blood glucose level estimate on a later day (*e.g.*, second or third day) of the testing period is assigned a higher confidence level than an interpolated overnight fasting blood glucose level estimate on an earlier day (*e.g.,* first day) of the testing period. The process 800 stops, at 810, when the confidence level is calculated.

The testing period is an infrequent, defined period of data capture. For example, the testing period is two or more days (*e.g*., three days). The testing period is initiated by the user. Additionally or alternatively, the testing period is pre-configured to start on a pre-determined date. In this case, the testing period is initiated by the user's mobile device. The mobile device indicates (*e.g.,* via display, reminder, or alert) to the user that the testing period has initiated.

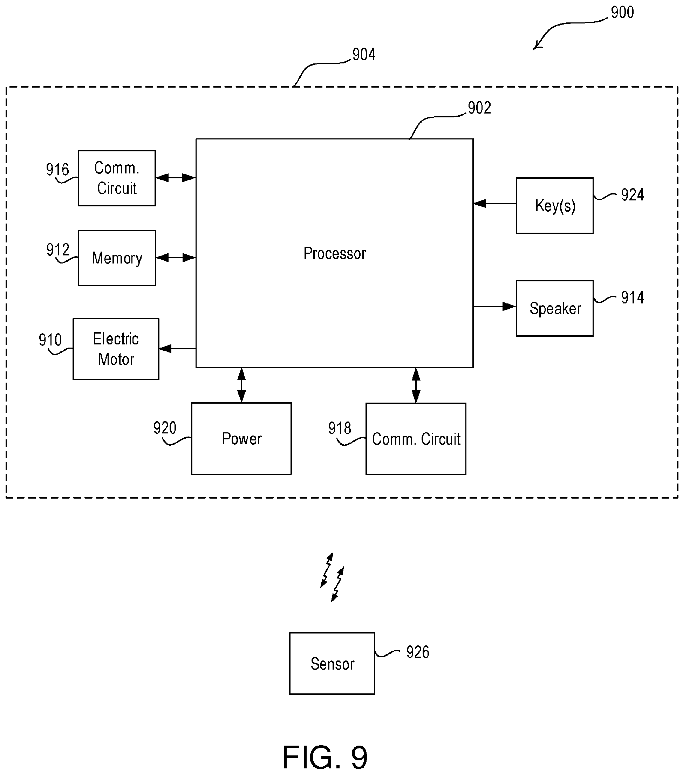

FIG. 9 is a block diagram of an example blood glucose monitoring device 900 (*e.g.,* such as the CGM 102 shown in FIG. 1). The blood glucose monitoring device 900 is a CGM or FGM, for example. The blood glucose monitoring device 900 includes a subcutaneous sensor 926 that is used to sense and monitor the amount of glucose in interstitial fluid of the user. Data is transmitted from the sensor 926 to a transmitting device 904. When the blood glucose monitoring device 900 is a CGM, the transmitting device 904 is located directly over the sensor 926 and wirelessly powers the data transfer from the sensor 926 via power supply 920. When the blood glucose monitoring device 900 is an FGM, the transmitting device 904 is a mobile device or other reader device that instantaneously receives the blood glucose information from the sensor 926 when the device is within the RF range of the sensor 926.

The transmitting device 904 receives data communications from the sensor 926 via a communication circuit 918. The communication circuit 918 is in electrical communication with a processor 902. The processor 902 includes one or more circuits, such as general-purpose processors, special purpose processors, conventional processors, digital signal processors (DSPs), microprocessors, integrated circuits, a programmable logic device (PLD), application specific integrated circuits (ASICs), or the like. The processor 902 performs signal coding, data processing, power control, input/output processing, or any other functionality that enables the transmitting device 904 to perform as described herein.

The transmitting device 904 includes another communication circuit 916 for communicating with other devices. The processor 902 is in electrical communication with the communication circuit 916 for sending or receiving information. The communication circuits 916, 918 are capable of performing wired or wireless communications. For example, the communication circuits 916, 918 includes one or more radio frequency (RF) transceivers for transmitting and receiving RF signals (*e.g*., BLUETOOTH^{®}, near field communication (NFC), WIFI^{®}, WI-MAX^{®}, cellular, or other suitable RF signals) via an antenna, or other communications module capable of performing wireless communications. The communication circuits 916, 918 communicate using the same RF protocol or a different RF protocol.

The processor 902 stores information in or retrieves information from the memory 912. The memory 912 includes a non-removable memory or a removable memory. The non-removable memory includes random-access memory (RAM), read-only memory (ROM), a hard disk, or any other type of non-removable memory storage. The removable memory includes a subscriber identity module (SIM) card, a memory stick, a memory card (*e.g.,* a digital camera memory card), or any other type of removable memory. The processor 902 accesses the memory 912 for executable instructions or other information that is used by the transmitting device 904. The processor 902 is in electrical communication with a one or more input keys 924 for providing input to the processor 902.

The processor 902 is in electrical communication with or controls a speaker 914. The speaker 914 provides an audible sound (*e.g*., tone, beep, or buzz) in response to a triggering event detected by the processor 902.

The blood glucose monitoring device 900 includes an electric motor 910 that is in electrical communication with or controlled by the processor 902. The electric motor 910 rotates and causes the blood glucose monitoring device 900 to vibrate (*e.g*., to indicate an alert) in response to a triggering event detected by the processor 902. The electric motor 910 provides an alert to supplement the audible alarm or replace the audible alarm provided by the speaker 914.

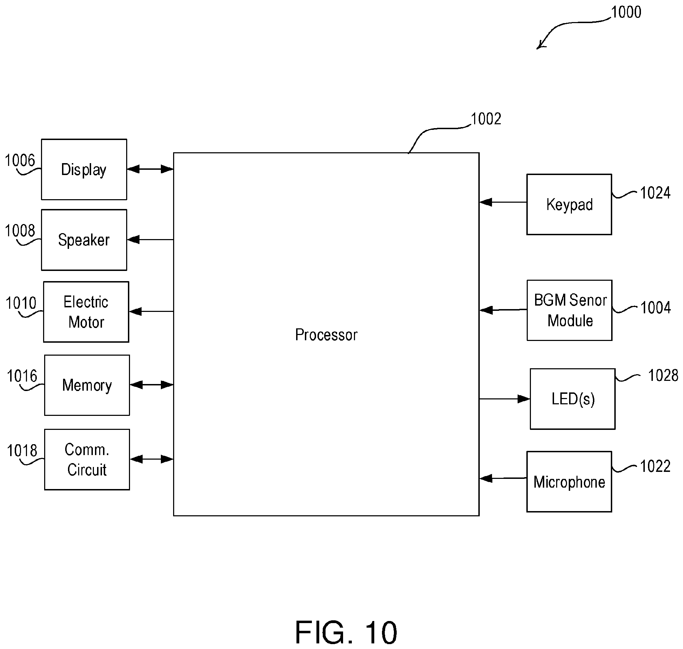

FIG. 10 is a block diagram of an example blood glucose measuring (BGM) device 1000 (*e.g.,* such as the BGM 106 shown in FIG. 1). The blood glucose monitoring device 1000 is a spot-monitoring blood glucose (SMBG) meter, for example. As shown in FIG. 10, the BGM device 1000 includes a processor 1002 for controlling the functionality of the BGM device 1000. In various embodiments, the processor 1002 includes one or more digital logic devices, such as general-purpose processors, special purpose processors, digital signal processors (DSPs), microprocessors, integrated circuits, programmable logic devices (PLDs), application specific integrated circuits (ASICs), and any other suitable digital logic device. The processor 1002 performs signal coding, data processing, power control, image processing, input/output processing, or any other functionality that enables the BGM device 1000 to perform as described herein.

The processor 1002 stores information in or retrieve information from the memory 1016. The memory 1016 includes a non-removable memory or a removable memory. The non-removable memory includes random-access memory (RAM), read-only memory (ROM), a hard disk, or any other type of non-removable memory storage. The removable memory includes a subscriber identity module (SIM) card, a memory stick, a memory card (*e.g.,* a digital camera memory card), or any other type of removable memory. The processor 1002 accesses the memory 1016 for executable instructions or other information that is used by the BGM device 1000.

The BGM device 1000 includes one or more communication circuits 1018. The processor 1002 is in electrical communication with the communication circuit 1018 for sending or receiving information. The communication circuit 1018 is capable of performing wired or wireless communications. For example, the communication circuit 1018 includes one or more radio frequency (RF) transceivers for transmitting and receiving RF signals (*e.g*., BLUETOOTH^{®}, near field communication (NFC), WIFI^{®}, WI-MAX^{®}, cellular, or other suitable RF signals) via an antenna, or other communications module capable of performing wireless communications. One or more communication circuits 1018 are capable of performing infrared (IR) communications.

The processor 1002 is in electrical communication with a keypad 1024 for providing input to the processor 1002. The keypad 1024 includes one or more keys for receiving input from a user. The keypad 1024 includes hard or soft keys for which the function of the keys may change as a user performs selections.

Other input into the processor 1002 is provided by the BGM sensor module 1004. The BGM sensor module 1004 includes a blood glucose measuring engine that analyzes blood samples provided by a patient on a blood glucose measurement strip and measures the amount of blood glucose in the samples.

The processor 1002 is in electrical communication with or generate images on a display 1006 for providing information to a user. The communication between the display 1006 and the processor 1002 is a two-way communication, as the display 1006 includes a touch screen module capable of receiving information from a user and providing such information to the processor 1002. For example, the display 1006 provides soft buttons for selection by a user that are recognized by the touch screen module and provided to the processor 1002 as input.

The processor 1002 is in electrical communication with or control a speaker 1008. The speaker 1008 provides an audible sound (*e.g*., tone, beep, or buzz) in response to a triggering event detected by the processor 1002.

The BGM device 1000 includes an electric motor 1010 that is in electrical communication with or controlled by the processor 1002. The electric motor 1010 rotates and causes the BGM device 1000 to vibrate (*e.g*., to indicate an alert) in response to a triggering event detected by the processor 1002. The electric motor 1010 provides an alert to supplement the audible alarm or replace the audible alarm provided by the speaker 1008.

The processor 1002 is in electrical communication with or receive information from a microphone 1022. For example, the processor 1002 receives audio signals via the microphone 1022.

The BGM device 1000 includes a visual indicator, such as one or more one or more light-emitting diodes (LEDs) 1028. One or more LEDs 1028 are illuminated or flashed to provide an alert or communicate other information to the user (*e.g.,* low battery or turning on of the device).

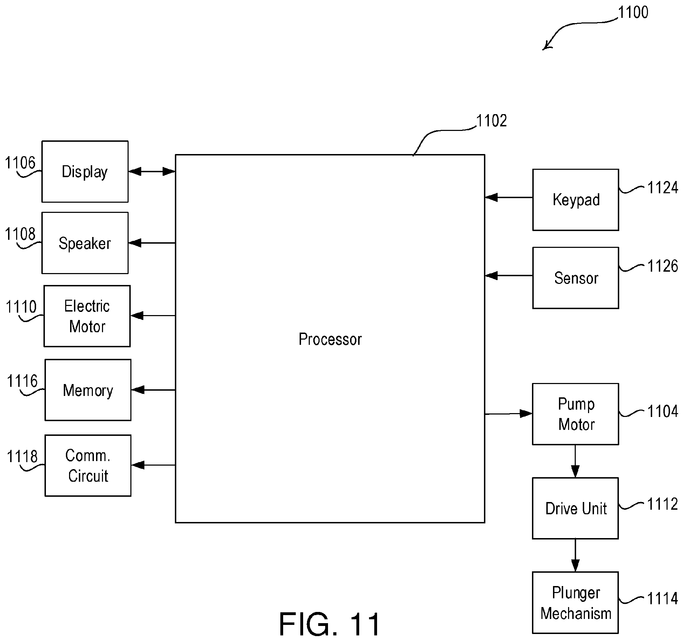

FIG. 11 is a block diagram illustrating an example of an insulin pump 1100 (*e.g.,* such as the ambulatory non-durable insulin pump 116 or the ambulatory durable insulin pump 118 shown in FIG. 1). As shown in FIG. 11, the insulin pump 1100 includes a processor 1102. The processor 1102 includes one or more circuits, such as general-purpose processors, special purpose processors, conventional processors, digital signal processors (DSPs), microprocessors, integrated circuits, a programmable logic device (PLD), application specific integrated circuits (ASICs), or the like. The processor 1102 performs signal coding, data processing, power control, image processing, input/output processing, or any other functionality that enables the insulin pump 1100 to perform as described herein.

In the embodiment of FIG. 11, the processor 1102 is in electrical communication with or control a pump motor 1104 in the insulin pump 1100. The pump motor 1104 drives a drive unit 1112 that pushes a plunger mechanism 1114. The plunger mechanism 1114 ejects insulin from an insulin cartridge (not shown). The insulin cartridge includes a supply of insulin for delivery to a user.

The processor 1102 is in electrical communication with or generate images on a display 1106 for providing information to a user. The communication between the display 1106 and the processor 1102 is a two-way communication, as the display 1106 includes a touch screen module capable of receiving information from a user and providing such information to the processor 1102. For example, the display 1106 provides soft buttons for selection by a user that are recognized by the touch screen module and provided to the processor 1102 as input.

The processor 1102 is in electrical communication with or control a speaker 1108. The speaker 1108 provides an audible sound (*e.g*., tone, beep, or buzz) in response to a triggering event detected by the processor 1102.

The insulin pump 1100 includes an electric motor 1110 that is in electrical communication with or controlled by the processor 1102. The electric motor 1110 rotates and causes the insulin pump to vibrate (*e.g*., to indicate an alert) in response to a triggering event detected by the processor 1102. The electric motor 1110 provides an alert to supplement the audible alarm or replace the audible alarm provided by the speaker 1108.

The processor 1102 is in electrical communication with a memory 1116. The processor stores information in or retrieves information from the memory 1116. The memory 1116 includes a non-removable memory or a removable memory for storing computer-readable media. The non-removable memory includes random-access memory (RAM), read-only memory (ROM), a hard disk, or any other type of non-removable memory storage. The removable memory includes a subscriber identity module (SIM) card, a memory stick, a memory card (*e.g.,* a digital camera memory card), or any other type of removable memory. The processor 1102 accesses the memory 1116 for executable instructions or other information that is used by the insulin pump 1100.

The insulin pump 1100 includes a communication circuit 1118. The processor 1102 is in electrical communication with the communication circuit 1118 for sending or receiving information. The communication circuit 1118 is capable of performing wired or wireless communications. For example, the wireless communications circuit 1118 includes a radio frequency (RF) transceiver for transmitting and receiving RF signals (*e.g*., BLUETOOTH^{®}, near field communication (NFC), WIFI^{®}, WI-MAX^{®}, cellular, or other suitable RF signals) via an antenna, or other communications module capable of performing wireless communications. The communication circuit 1118 is capable of performing infrared (IR) communications.

The processor 1102 is in electrical communication with a keypad 1124 for providing input to the processor 1102. The keypad 1124 includes one or more keys for receiving input from a user. The keypad 1124 includes hard or soft keys for which the function of the keys may change as a user performs selections.

Other input into the processor 1102 is provided by sensors 1126. The sensors 1126 includes a pressure sensor that is sensitive to the pressure within a reservoir of insulin; a cartridge sensor that is sensitive to the presence of an insulin cartridge, or a motion sensor that detects the motion of a gear (not shown) in the drive unit 1112.

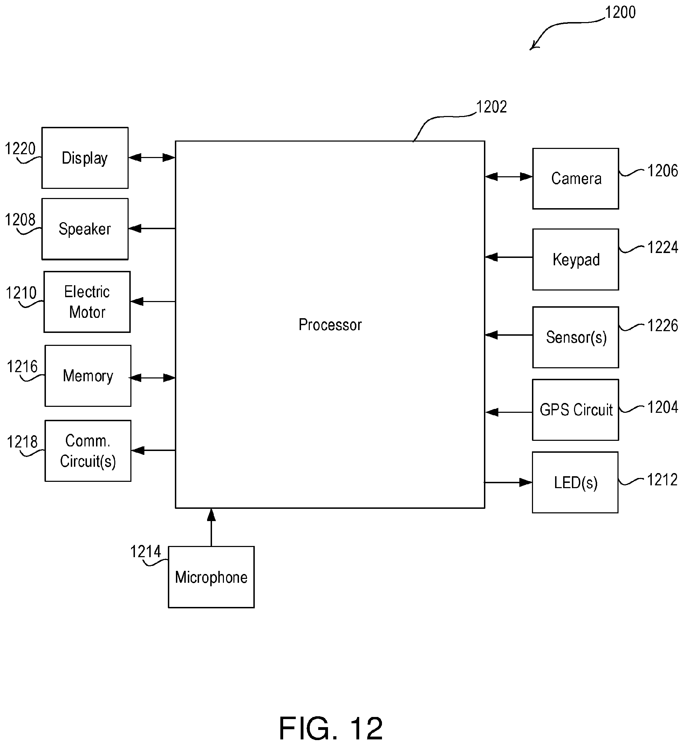

FIG. 12 is a block diagram of an example computing device 1200 (*e.g.,* such as the mobile device 104 shown in FIG. 1). The computing device is a mobile computing device, such as a tablet, a cellular phone, a wearable device, a CGM controller device, or another computing device, for example. As shown in FIG. 12, the computing device 1200 includes a processor 1202 for controlling the functionality of the computing device 1200. The processor 1202 includes one or more circuits, such as general-purpose processors, special purpose processors, conventional processors, digital signal processors (DSPs), microprocessors, integrated circuits, a programmable logic device (PLD), application specific integrated circuits (ASICs), or the like. The processor 1202 performs signal coding, data processing, power control, image processing, input/output processing, or any other functionality that enables the computing device 1200 to perform as described herein.

The processor 1202 stores information in or retrieve information from the memory 1216. The memory 1216 includes a non-removable memory or a removable memory. The non-removable memory includes random-access memory (RAM), read-only memory (ROM), a hard disk, or any other type of non-removable memory storage. The removable memory includes a subscriber identity module (SIM) card, a memory stick, a memory card (*e.g.,* a digital camera memory card), or any other type of removable memory. The processor 1202 accesses the memory 1216 for executable instructions or other information that is used by the computing device 1200.

The computing device 1200 includes a camera 1206 that is in communication with the processor 1202. The camera 1206 is a digital camera or other optical device capable of generating images or videos (*e.g*., image sequences) for being captured at the computing device 1200. The camera 1206 includes a lighting device capable of flashing to in response to signals from the processor 1202. The lighting device flashes to provide alerts via the camera 1206.

The computing device 1200 includes one or more communication circuits 1218. The processor 1202 is in electrical communication with the communication circuit 1218 for sending or receiving information. The communication circuit 1218 is capable of performing wired or wireless communications. For example, the communication circuit 1218 includes one or more radio frequency (RF) transceivers for transmitting and receiving RF signals (*e.g*., BLUETOOTH^{®}, near field communication (NFC), WIFI^{®}, WI-MAX^{®}, cellular, or other suitable wireless transceivers) via an antenna, or other communications module capable of performing wireless communications. One or more communication circuits 1218 are capable of performing infrared (IR) communications.

The processor 1202 is in electrical communication with a keypad 1224 for providing input to the processor 1202. The keypad 1224 includes one or more keys for receiving input from a user. The keypad 1224 includes hard or soft keys for which the function of the keys may change as a user performs selections.

Other input into the processor 1202 is provided by one or more sensors 1226. The sensors 1226 includes a motion sensor, a proximity sensor, a heartrate monitoring sensor, an accelerometer, a gyroscope, or another sensor on the computing device. The motion sensor transmits infrared signals or use image processing to sense movement. The proximity sensor transmits infrared signals to detect when an object is within a predefined proximity. The heartrate monitoring sensor implements photoplethysmography to detect the amount of blood flow in the user. The heartrate monitoring sensor includes one or more LED or photodiodes to detect the amount of blood flow in the user. The heartrate monitoring sensor implements infrared technology to detect the amount of blood flow in the user. The heartrate monitoring sensor takes an electrocardiogram (ECG) and detects information about the user's heartrate from the ECG. The accelerometer measures the non-gravitational acceleration of the computing device 1200 in a given direction. The accelerometer responds to vibrations associated with movement in a given direction. The measurements from the accelerometer are used by the processor 1202 to determine the magnitude or direction of the relative movement of the computing device 1200, or the user's relative position (e.g., standing, sitting, or lying down). The gyroscope is used to determine the orientation of the computing device 1200.

The processor 1202 is in electrical communication with or generate images on a display 1220 for providing information to a user. The communication between the display 1220 and the processor 1202 is a two-way communication, as the display 1220 includes a touch screen module capable of receiving information from a user and providing such information to the processor 1202. For example, the display 1220 provides soft buttons for selection by a user that are recognized by the touch screen module and provided to the processor 1202 as input.

The processor 1202 is in electrical communication with or control a speaker 1208. The speaker 1208 provides an audible sound (e.g., tone, beep, or buzz) in response to a triggering event detected by the processor 1202.

The computing device 1200 includes an electric motor 1210 that is in electrical communication with or controlled by the processor 1202. The electric motor 1210 rotates and causes the computing device 1200 to vibrate (e.g., to indicate an alert) in response to a triggering event detected by the processor 1202. The electric motor 1210 provides an alert to supplement the audible alarm or replace the audible alarm provided by the speaker 1208.

The processor 1202 is in electrical communication with or receive information from a microphone 1214. For example, the processor 1202 receives audio signals via the microphone 1214.

The computing device 1200 includes a global positioning system (GPS) circuit 1204. The GPS circuit 1204 is capable of receiving GPS information. The processor 1202 is capable of determining the GPS coordinates (*e.g*., latitude and longitude) of the computing device 1200 based on the GPS information received via the GPS circuit.

The computing device 1200 includes a visual indicator, such as one or more one or more light-emitting diodes (LEDs) 1212. One or more LEDs 1212 are illuminated or flashed to provide an alert or communicate other information to the user (*e.g.,* low battery or turning on of the device).

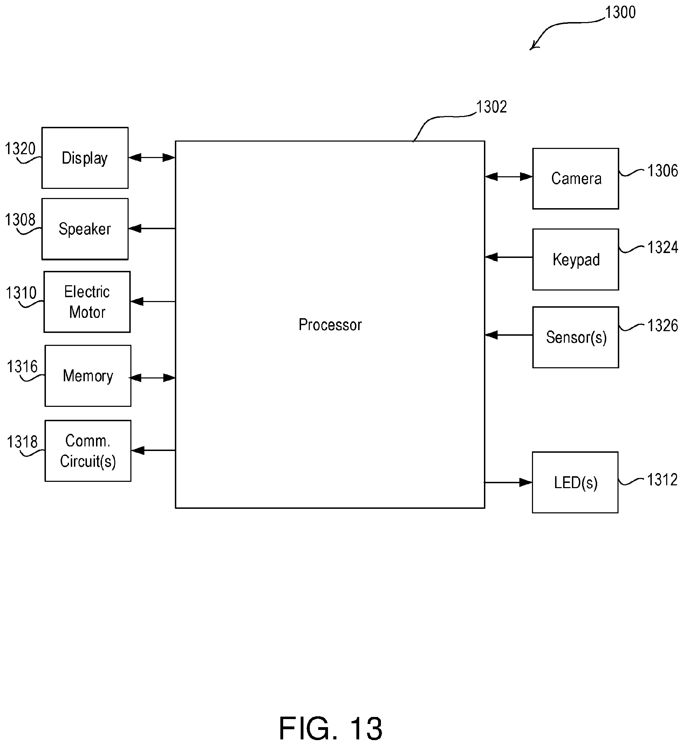

FIG. 13 is a block diagram of an example smart plate 1300 (*e.g.,* such as the smart plate 128 shown in FIG. 1). The smart plate is configured to automate meal consumption tracking for the user, for example, during a structured testing period. As shown in FIG. 13, the smart plate 1300 includes a processor 1302 for controlling the functionality of the smart plate 1300. The processor 1302 includes one or more circuits, such as general-purpose processors, special purpose processors, conventional processors, digital signal processors (DSPs), microprocessors, integrated circuits, a programmable logic device (PLD), application specific integrated circuits (ASICs), or the like. The processor 1302 performs signal coding, data processing, power control, image processing, input/output processing, or any other functionality that enables the smart plate 1300 to perform as described herein.

The processor 1302 stores information in or retrieve information from the memory 1316. The memory 1316 includes a non-removable memory or a removable memory. The non-removable memory includes random-access memory (RAM), read-only memory (ROM), a hard disk, or any other type of non-removable memory storage. The removable memory includes a subscriber identity module (SIM) card, a memory stick, a memory card *(e.g.,* a digital camera memory card), or any other type of removable memory. The processor 1302 accesses the memory 1316 for executable instructions or other information that is used by the smart plate 1300.

The smart plate 1300 includes a camera 1306 that is in communication with the processor 1302. The camera 1306 is a digital camera or other optical device capable of generating images or videos (*e.g*., image sequences) for being captured at the smart plate 1300. The camera 1306 includes a lighting device capable of flashing to in response to signals from the processor 1302. The lighting device flashes to provide alerts via the camera 1306. The camera 1306 is configured to read a machine readable optical label (*e.g.,* a bar code, a QR code, or some other unique identifier) on a pre-packaged meal (*e.g*., such as the pre-packaged meals 130 shown in FIG. 1) or a packaged food product. The images captured by the camera 1306 are used by the processor 1302 to determine nutrition information for the pre-packaged meal or packaged food product.

The smart plate 1300 includes one or more communication circuits 1318. The processor 1302 is in electrical communication with the communication circuit 1318 for sending or receiving information. The communication circuit 1318 is capable of performing wired or wireless communications. For example, the communication circuit 1318 includes one or more radio frequency (RF) transceivers for transmitting and receiving RF signals (e.g., BLUETOOTH^{®}, near field communication (NFC), WIFI^{®}, WI-MAX^{®}, cellular, or other suitable wireless transceivers) via an antenna, or other communications module capable of performing wireless communications. One or more communication circuits 1318 are capable of performing infrared (IR) communications.

The processor 1302 is in electrical communication with a keypad 1324 for providing input to the processor 1302. The keypad 1324 includes one or more keys for receiving input from a user. The keypad 1324 includes hard or soft keys for which the function of the keys may change as a user performs selections.

Other input into the processor 1302 is provided by one or more sensors 1326. The sensors 1326 includes a motion sensor, a proximity sensor, a weight sensor, an accelerometer, a gyroscope, or another sensor on the smart plate. The motion sensor transmits infrared signals or use image processing to sense movement. The proximity sensor transmits infrared signals to detect when an object is within a predefined proximity. The weight sensor measure a weight of food on the smart plate 1300. The measurements from the weight sensor are used by the processor 1302 to determine a weight of a meal, a weight of each food in the meal, a percentage of the meal that the user consumed. The accelerometer measures the non-gravitational acceleration of the smart plate 1300 in a given direction. The accelerometer responds to vibrations associated with movement in a given direction. The measurements from the accelerometer are used by the processor 1302 to determine the magnitude or direction of the relative movement of the smart plate 1300, or the user's relative position (e.g., standing, sitting, or lying down). The gyroscope is used to determine the orientation of the smart plate 1300.

The processor 1302 is in electrical communication with or generate images on a display 1320 for providing information to a user. The communication between the display 1320 and the processor 1302 is a two-way communication, as the display 1320 includes a touch screen module capable of receiving information from a user and providing such information to the processor 1302. For example, the display 1320 provides soft buttons for selection by a user that are recognized by the touch screen module and provided to the processor 1302 as input.

The processor 1302 is in electrical communication with or control a speaker 1308. The speaker 1308 provides an audible sound (*e.g*., tone, beep, or buzz) in response to a triggering event detected by the processor 1302.

The smart plate 1300 includes an electric motor 1310 that is in electrical communication with or controlled by the processor 1302. The electric motor 1310 rotates and causes the smart plate 1300 to vibrate (*e.g*., to indicate an alert) in response to a triggering event detected by the processor 1302. The electric motor 1310 provides an alert to supplement the audible alarm or replace the audible alarm provided by the speaker 1308.

The smart plate 1300 includes a global positioning system (GPS) circuit 1304. The GPS circuit 1304 is capable of receiving GPS information. The processor 1302 is capable of determining the GPS coordinates (e.g., latitude and longitude) of the smart plate 1300 based on the GPS information received via the GPS circuit.

The smart plate 1300 includes a visual indicator, such as one or more one or more light-emitting diodes (LEDs) 1312. One or more LEDs 1312 are illuminated or flashed to provide an alert or communicate other information to the user (*e.g.,* low battery or turning on of the device).

The methods described herein are implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random-access memory (RAM), removable disks, and optical media such as CD-ROM disks, and digital versatile disks (DVDs).

## Claims

1. A computer-implemented method comprising:
receiving blood glucose data from a blood glucose monitor (106, 900, 1000), the blood glucose data comprising a plurality of blood glucose levels of a user (100) that are measured during a testing period;
receiving testing period data associated with the user (100) during the testing period, wherein the testing period data enables one or more of disease progression analysis or therapy analysis at an end of the testing period, the testing period data comprising:
dietary intake data associated with a plurality of meals consumed by the user (100) during the testing period,
medication data associated with a plurality of medication doses taken by the user (100) during the testing period, and
activity data associated with a plurality of pre-scheduled activities for the user (100) during the testing period;
determining that a portion of the testing period data is missing based on an analysis of the received data;
imputing the missing portion of the testing period data with substitute dietary intake data in response to determining that the missing portion of the testing period data comprises dietary intake data associated with a meal of the plurality of meals and determining that the user (100) consumed the meal;
imputing the missing portion of the testing period data with substitute medication data in response to determining that the missing portion of data comprises medication data associated with a medication dose of the plurality of medication doses and determining that the user (100) took the medication dose;
imputing the missing portion of the testing period data with substitute activity data in response to determining that the missing portion of data comprises activity data associated with a pre-scheduled activity of the plurality of pre-scheduled activities and determining that the user (100) participated in the pre-scheduled activity;
calculating a confidence level associated with the substitute dietary intake data, the substitute medication data, or the substitute activity data;
adding the substitute dietary intake data, the substitute medication data, or the substitute activity data to the testing period data when the confidence level is greater than a pre-defined threshold;
extending the testing period in response to one or more of:
determining that the user (100) did not consume the meal based on an analysis of one or more of the plurality of glucose levels proximate to a first time period associated with the meal,
determining that the user (100) did not take the medication dose based on an analysis of one or more of the plurality of glucose levels proximate to a second time period associated with the medication dose,
determining that the user (100) did not participate in the pre-scheduled activity based on an analysis of one or more of the plurality of glucose levels proximate to a third time period associated with the pre-scheduled activity, or
determining that the confidence level is below a pre-determined confidence threshold;
wherein the testing period comprises a plurality of days, and wherein the substitute dietary intake data, the substitute activity data, and the substitute medication data are based on a generic population-based blood glucose model in response to the missing portion of the testing period data being associated with a first time period on a first day of the plurality of days, the generic population-based model including predictive blood glucose level responses using data from persons having a similar profile to the user (100); and
wherein the substitute dietary intake data, the substitute activity data, and the substitute medication data are based on a time-weighted blood glucose model developed using blood glucose data of the user (100) received on the first day of the plurality of days in response to the missing portion of the testing period data being associated with a second time period on a second or third day of the plurality of days, the time-weighted blood glucose model including predictive blood glucose level responses using the testing period data from previous days of the testing period.

2. The method of claim 1, wherein the analysis of the received testing period data comprises:
an analysis of one or more blood glucose levels of the plurality of glucose levels; and
an analysis of the dietary intake data, the medication data, and the activity data.

3. The method of claim 2, wherein the analysis of the one or more blood glucose levels comprises determining that a blood glucose level is greater than a pre-defined blood glucose threshold.

4. The method of claim 3, wherein the pre-defined blood glucose threshold is determined based on an expected blood glucose level associated with one or more of a carbohydrate content of the meal, a glycemic profile of the meal, a dynamic fingerprint associated with a medicine of the medication dose, a type of the pre-scheduled activity, a length of the pre-scheduled activity, or a pre-determined blood glucose level increase associated with the user (100).

5. The method of claim 1, wherein the testing period comprises a plurality of days, and wherein the pre-defined blood glucose threshold is determined based on a generic population-based blood glucose model in response to the meal, pre-scheduled activity, or medication dose being on a first day of the plurality of days.

6. The method of claim 5, wherein the pre-defined blood glucose threshold is determined based on a time-weighted blood glucose model in response to the meal, pre-scheduled activity, or medication dose being on a second or third day of the plurality of days, the time-weighted blood glucose model developed using blood glucose data of the user (100) received on the first day of the plurality of days.

7. The method of claim 1, wherein the blood glucose monitor (106, 900, 1000) is a continuous glucose monitor (CGM) (102) or a spot-monitoring blood glucose (SMBG) meter.

8. The method of claim 1, wherein determining that the missing portion of the testing period data comprises dietary intake data comprises analysis of one or more scheduled meals, and wherein determining that the missing portion of data comprises medication data comprises analysis of one or more scheduled medication doses, and wherein determining that the missing portion of the testing period data comprises activity data comprises analysis of one or more pre-scheduled activities.

9. The method of claim 1, wherein the confidence levels for each instance of substitute data imputation on a day of the testing period are aggregated to determine a daily aggregate confidence level, and wherein the testing period is extended in response to the daily aggregate confidence level being below the pre-defined confidence threshold.

10. The method of claim 1, further comprising:
identifying, using the substitute dietary intake data, the substitute medication data, or the substitute activity data, a progression or a regression in a diabetic condition associated with the user (100); and
adjusting a configuration of an insulin pump to increase or reduce the supply of insulin to the user (100) in response to the progression or the regression in the diabetic condition.

11. The method of claim 1, further comprising updating a learning model associated with the user (100) using the testing period data.

## Patentansprüche

1. Computerimplementiertes Verfahren, umfassend:
Empfangen von Blutzuckerdaten von einem Blutzuckermonitor (106, 900, 1000), wobei die Blutzuckerdaten eine Vielzahl von Blutzuckerspiegeln eines Benutzers (100) umfassen, die während einer Testperiode gemessen werden;
Empfangen von Testperiodendaten, die dem Benutzer (100) zugeordnet sind, während der Testperiode, wobei die Testperiodendaten eine oder mehrere von einer Krankheitsprogressionsanalyse oder einer Therapieanalyse an einem Ende der Testperiode ermöglichen, wobei die Testperiodendaten umfassen:
Nahrungsaufnahmedaten, die einer Vielzahl von Mahlzeiten zugeordnet sind, die von dem Benutzer (100) während der Testperiode konsumiert werden,
Medikationsdaten, die einer Vielzahl von Medikationsdosen zugeordnet sind, die von dem Benutzer (100) während der Testperiode eingenommen werden, und
Aktivitätsdaten, die einer Vielzahl von vorab geplanten Aktivitäten für den Benutzer (100) während der Testperiode zugeordnet sind;
Bestimmen, dass ein Teil der Testperiodendaten fehlt, basierend auf einer Analyse der empfangenen Daten;
Zuschreiben des fehlenden Teils der Testperiodendaten zu Ersatznahrungsaufnahmedaten als Reaktion auf das Bestimmen, dass der fehlende Teil der Testperiodendaten Nahrungsaufnahmedaten umfasst, die einer Mahlzeit der Vielzahl von Mahlzeiten zugeordnet sind, und Bestimmen, dass der Benutzer (100) die Mahlzeit konsumiert hat;
Zuschreiben des fehlenden Teils der Testperiodendaten zu Ersatzmedikationsdaten als Reaktion auf das Bestimmen, dass der fehlende Teil der Daten Medikationsdaten umfasst, die einer Medikationsdosis der Vielzahl von Medikationsdosen zugeordnet sind, und Bestimmen, dass der Benutzer (100) die Medikationsdosis eingenommen hat;
Zuschreiben des fehlenden Teils der Testperiodendaten zu Ersatzaktivitätsdaten als Reaktion auf das Bestimmen, dass der fehlende Teil der Daten Aktivitätsdaten umfasst, die einer vorab geplanten Aktivität der Vielzahl von vorab geplanten Aktivitäten zugeordnet sind, und Bestimmen, dass der Benutzer (100) an der vorab geplanten Aktivität teilgenommen hat;
Berechnen eines Konfidenzniveaus, das den Ersatznahrungsaufnahmedaten, den Ersatzmedikationsdaten oder den Ersatzaktivitätsdaten zugeordnet ist;
Hinzufügen der Ersatznahrungsaufnahmedaten, der Ersatzmedikationsdaten oder der Ersatzaktivitätsdaten zu den Testperiodendaten, wenn das Konfidenzniveau höher als ein vordefinierter Schwellenwert ist;
Verlängern der Testperiode als Reaktion auf eines oder mehrere von Folgendem:
Bestimmen, dass der Benutzer (100) die Mahlzeit nicht konsumiert hat, basierend auf einer Analyse von einem oder mehreren der Vielzahl von Zuckerspiegeln in der Nähe einer ersten Zeitperiode, die der Mahlzeit zugeordnet ist,
Bestimmen, dass der Benutzer (100) die Medikationsdosis nicht eingenommen hat, basierend auf einer Analyse von einem oder mehreren der Vielzahl von Zuckerspiegeln in der Nähe einer zweiten Zeitperiode, die der Medikationsdosis zugeordnet ist,
Bestimmen, dass der Benutzer (100) nicht an der vorab geplanten Aktivität teilgenommen hat, basierend auf einer Analyse von einem oder mehreren der Vielzahl von Zuckerspiegeln in der Nähe einer dritten Zeitperiode, die der vorab geplanten Aktivität zugeordnet ist, oder
Bestimmen, dass das Konfidenzniveau unter einem vorbestimmten Konfidenzschwellenwert liegt;
wobei die Testperiode eine Vielzahl von Tagen umfasst und wobei die Ersatznahrungsaufnahmedaten, die Ersatzaktivitätsdaten und die Ersatzmedikationsdaten auf einem generischen populationsbasierten Blutzuckermodell als Reaktion darauf basieren, dass der fehlende Teil der Testperiodendaten einer ersten Zeitperiode an einem ersten Tag der Vielzahl von Tagen zugeordnet ist, wobei das generische populationsbasierte Modell prädiktive Blutzuckerspiegelreaktionen unter Verwendung von Daten von Personen mit einem ähnlichen Profil wie der Benutzer (100) einschließt; und
wobei die Ersatznahrungsaufnahmedaten, die Ersatzaktivitätsdaten und die Ersatzmedikationsdaten auf einem zeitgewichteten Blutzuckermodell basieren, das unter Verwendung von Blutzuckerdaten des Benutzers (100) entwickelt wurde, die an dem ersten Tag der Vielzahl von Tagen als Reaktion darauf empfangen wurden, dass der fehlende Teil der Testperiodendaten einer zweiten Zeitperiode an einem zweiten oder dritten Tag der Vielzahl von Tagen zugeordnet ist, wobei das zeitgewichtete Blutzuckermodell prädiktive Blutzuckerspiegelreaktionen unter Verwendung der Testperiodendaten von vorherigen Tagen der Testperiode einschließt.

2. Verfahren nach Anspruch 1, wobei die Analyse der empfangenen Testperiodendaten umfasst:
eine Analyse von einem oder mehreren Blutzuckerspiegeln der Vielzahl von Zuckerspiegeln; und
eine Analyse der Nahrungsaufnahmedaten, der Medikationsdaten und der Aktivitätsdaten.

3. Verfahren nach Anspruch 2, wobei die Analyse des einen oder der mehreren Blutzuckerspiegel das Bestimmen umfasst, dass ein Blutzuckerspiegel höher als ein vordefinierter Blutzuckerschwellenwert ist.

4. Verfahren nach Anspruch 3, wobei der vordefinierte Blutzuckerschwellenwert basierend auf einem erwarteten Blutzuckerspiegel bestimmt wird, der einem oder mehreren von einem Kohlenhydratgehalt der Mahlzeit, einem glykämischen Profil der Mahlzeit, einem dynamischen Fingerabdruck, der einem Medikament der Medikationsdosis zugeordnet ist, einem Typ der vorab geplanten Aktivität, einer Länge der vorab geplanten Aktivität oder einer vorbestimmten Blutzuckerspiegelerhöhung, die dem Benutzer (100) zugeordnet ist, zugeordnet ist.

5. Verfahren nach Anspruch 1, wobei die Testperiode eine Vielzahl von Tagen umfasst und wobei der vordefinierte Blutzuckerschwellenwert basierend auf einem generischen populationsbasierten Blutzuckermodell als Reaktion darauf bestimmt wird, dass die Mahlzeit, die vorab geplante Aktivität oder die Medikationsdosis an einem ersten Tag der Vielzahl von Tagen erfolgt.

6. Verfahren nach Anspruch 5, wobei der vordefinierte Blutzuckerschwellenwert basierend auf einem zeitgewichteten Blutzuckermodell als Reaktion darauf bestimmt wird, dass die Mahlzeit, die vorab geplante Aktivität oder die Medikationsdosis an einem zweiten oder dritten Tag der Vielzahl von Tagen erfolgt, wobei das zeitgewichtete Blutzuckermodell unter Verwendung von Blutzuckerdaten des Benutzers (100) entwickelt wurde, die an dem ersten Tag der Vielzahl von Tagen empfangen wurden.

7. Verfahren nach Anspruch 1, wobei der Blutzuckermonitor (106, 900, 1000) ein kontinuierlicher Glucosemonitor (CGM) (102) oder ein Spotmonitoring-Blutzuckermessgerät (SMBG) ist.

8. Verfahren nach Anspruch 1, wobei das Bestimmen, dass der fehlende Teil der Testperiodendaten Nahrungsaufnahmedaten umfasst, die Analyse einer oder mehrerer geplanter Mahlzeiten umfasst und wobei das Bestimmen, dass der fehlende Teil der Daten Medikationsdaten umfasst, die Analyse einer oder mehrerer geplanter Medikationsdosen umfasst und wobei das Bestimmen, dass der fehlende Teil der Testperiodendaten Aktivitätsdaten umfasst, die Analyse einer oder mehrerer vorab geplanter Aktivitäten umfasst.

9. Verfahren nach Anspruch 1, wobei die Konfidenzniveaus für jede Instanz der Ersatzdatenzuschreibung an einem Tag der Testperiode aggregiert werden, um ein tägliches aggregiertes Konfidenzniveau zu bestimmen, und wobei die Testperiode als Reaktion darauf verlängert wird, dass das tägliche aggregierte Konfidenzniveau unter dem vordefinierten Konfidenzschwellenwert liegt.

10. Verfahren nach Anspruch 1, ferner umfassend:
Identifizieren, unter Verwendung der Ersatznahrungsaufnahmedaten, der Ersatzmedikationsdaten oder der Ersatzaktivitätsdaten, einer Progression oder einer Regression bei einem diabetischen Zustand, der dem Benutzer (100) zugeordnet ist; und
Anpassen einer Konfiguration einer Insulinpumpe, um die Zufuhr von Insulin zu dem Benutzer (100) als Reaktion auf die Progression oder die Regression bei dem diabetischen Zustand zu erhöhen oder zu verringern.

11. Verfahren nach Anspruch 1, ferner umfassend das Aktualisieren eines Lemmodells, das dem Benutzer (100) zugeordnet ist, unter Verwendung der Testperiodendaten.

## Revendications

1. Procédé mis en œuvre par ordinateur comprenant :
la réception de données de glycémie en provenance d'un glucomètre (106, 900, 1000), les données de glycémie comprenant une pluralité de taux de glycémie d'un utilisateur (100) qui sont mesurés pendant une période de test ;
la réception de données de la période de test associées à l'utilisateur (100) pendant la période de test, les données de la période de test permettant une ou plusieurs parmi une analyse de progression de maladie ou une analyse thérapeutique à la fin de la période de test, les données de la période de test comprenant :
des données d'apport alimentaire associées à une pluralité de repas consommés par l'utilisateur (100) pendant la période de test,
des données de médicament associées à une pluralité de doses de médicament prises par l'utilisateur (100) pendant la période de test, et
des données d'activité associées à une pluralité d'activités pré-planifiées pour l'utilisateur (100) pendant la période de test ;
la détermination qu'une partie des données de la période de test est manquante sur la base d'une analyse des données reçues ;
l'imputation de la partie manquante des données de la période de test avec des données d'apport alimentaire de substitution en réponse à la détermination que la partie manquante des données de la période de test comprend des données d'apport alimentaire associées à un repas de la pluralité de repas et la détermination que l'utilisateur (100) a consommé le repas ;
l'imputation de la partie manquante des données de la période de test avec des données de médicament de substitution en réponse à la détermination que la partie manquante des données comprend des données de médicament associées à une dose de médicament de la pluralité de doses de médicament et la détermination que l'utilisateur (100) a pris la dose de médicament ;
l'imputation de la partie manquante des données de la période de test avec des données d'activité de substitution en réponse à la détermination que la partie manquante des données comprend des données d'activité associées à une activité pré-planifiée de la pluralité d'activités pré-planifiées et la détermination que l'utilisateur (100) a participé à l'activité pré-planifiée ;
le calcul d'un niveau de confiance associé aux données d'apport alimentaire de substitution, aux données de médicament de substitution ou aux données d'activité de substitution ;
l'ajout des données d'apport alimentaire de substitution, des données de médicament de substitution ou des données d'activité de substitution aux données de la période de test lorsque le niveau de confiance est supérieur à un seuil prédéfini ;
l'allongement de la période de test en réponse à une ou plusieurs parmi :
la détermination que l'utilisateur (100) n'a pas consommé le repas sur la base d'une analyse d'un ou plusieurs de la pluralité de taux de glucose au voisinage d'une première période de temps associée au repas,
la détermination que l'utilisateur (100) n'a pas pris la dose de médicament sur la base d'une analyse d'un ou plusieurs de la pluralité de taux de glucose au voisinage d'une deuxième période de temps associée à la dose de médicament,
la détermination que l'utilisateur (100) n'a pas participé à l'activité pré-planifiée sur la base d'une analyse d'un ou plusieurs de la pluralité de taux de glucose au voisinage d'une troisième période associée à l'activité pré-planifiée, ou
la détermination que le niveau de confiance est au-dessous d'un seuil de confiance prédéterminé ;
dans lequel la période de test comprend une pluralité de jours, et dans lequel les données d'apport alimentaire de substitution, les données d'activité de substitution et les données de médicament de substitution sont basées sur un modèle de glycémie basé sur une population générique en réponse à la partie manquante des données de la période de test associées à une première période de temps pendant un premier jour de la pluralité de jours, le modèle basé sur une population générique incluant les réponses de taux de glycémie prédictives à l'aide des données provenant de personnes présentant un profil similaire à celui de l'utilisateur (100) ; et
dans lequel les données d'apport alimentaire de substitution, les données d'activité de substitution et les données de médicament de substitution sont basées sur un modèle de glycémie pondéré dans le temps développé à l'aide des données de glycémie de l'utilisateur (100) reçues pendant le premier jour de la pluralité de jours en réponse à la partie manquante des données de la période de test associées à une deuxième période de temps pendant un deuxième ou un troisième jour de la pluralité de jours, le modèle de glycémie pondéré dans le temps incluant des réponses de taux de glycémie prédictives à l'aide des données de la période de test des jours précédents de la période de test.

2. Procédé selon la revendication 1, dans lequel l'analyse des données de la période de test reçues comprend :
une analyse d'un ou plusieurs taux de glycémie de la pluralité de taux de glycémie ; et
une analyse des données d'apport alimentaire, des données de médicament et des données d'activité.

3. Procédé selon la revendication 2, dans lequel l'analyse du ou des taux de glycémie comprend la détermination qu'un taux de glycémie est supérieur à un seuil de glycémie prédéfini.

4. Procédé selon la revendication 3, dans lequel le seuil de glycémie prédéfini est déterminé sur la base d'un taux de glycémie attendu associé à un ou plusieurs parmi une teneur en glucides du repas, un profil glycémique du repas, une empreinte dynamique associée à un médicament de la dose de médicament, un type d'activité pré-planifiée, une durée de l'activité pré-planifiée ou une augmentation du taux de glycémie prédéterminé associée à l'utilisateur (100).

5. Procédé selon la revendication 1, dans lequel la période de test comprend une pluralité de jours, et dans lequel le seuil de glycémie prédéfini est déterminé sur la base d'un modèle de glycémie basé sur une population générique en réponse au repas, à l'activité pré-planifiée ou à la dose de médicament pendant un premier jour de la pluralité de jours.

6. Procédé selon la revendication 5, dans lequel le seuil de glycémie prédéfini est déterminé sur la base d'un modèle de glycémie pondérée dans le temps en réponse au repas, à l'activité pré-planifiée ou à la dose de médicament pendant un deuxième ou un troisième jour de la pluralité de jour, le modèle de glycémie pondérée dans le temps étant développé à l'aide des données de glycémie de l'utilisateur (100) reçues pendant le premier jour de la pluralité de jours.

7. Procédé selon la revendication 1, dans lequel le glucomètre (106, 900, 1000) est un moniteur de glucose en continu (CGM) (102) ou un lecteur d'auto-surveillance de la glycémie (SMBG).

8. Procédé selon la revendication 1, dans lequel la détermination que la partie manquante des données de la période de test comprend des données d'apport alimentaire comprend l'analyse d'un ou plusieurs repas planifiés, et dans lequel la détermination que la partie manquante des données comprend des données de médicament comprend l'analyse d'une ou plusieurs doses de médicament planifiées, et dans lequel la détermination que la partie manquante de données de la période de test comprend des données d'activité comprend l'analyse d'une ou plusieurs activités pré-planifiées.

9. Procédé selon la revendication 1, dans lequel les niveaux de confiance pour chaque cas d'imputation de données de substitution pendant un jour de la période de test sont agrégés pour déterminer un niveau de confiance agrégé quotidien, et dans lequel la période de test est prolongée en réponse au niveau de confiance agrégé quotidien au-dessous du seuil de confiance prédéfini.

10. Procédé selon la revendication 1, comprenant en outre :
l'identification, à l'aide des données d'apport alimentaire de substitution, des données de médicament de substitution ou des données d'activité de substitution, d'une progression ou d'une régression d'une condition diabétique associée à l'utilisateur (100) ; et
le réglage d'une configuration d'une pompe à insuline pour augmenter ou réduire l'administration d'insuline à l'utilisateur (100) en réponse à la progression ou à la régression de la condition diabétique.

11. Procédé selon la revendication 1, comprenant en outre la mise à jour d'un modèle d'apprentissage associé à l'utilisateur (100) à l'aide des données de la période de test.
